# EUROPEAN PATENT SPECIFICATION

(11) **EP 3 305 782 B1**
(45) Date of publication and mention of the grant of the patent: **30.04.2025**
(21) Application number: 17192451.7
(22) Date of filing: 21.09.2017
(51) Int. Cl.: C07D 405/12, C07D 409/12, C07D 487/04, C07D 307/91, C09K 11/06

(54) **ORGANIC COMPOUND AND ORGANIC LIGHT-EMITTING DIODE COMPRISING SAME**
ORGANISCHE VERBINDUNG UND ORGANISCHE LICHT-EMITTIERENDE DIODE DIESE ENTHALTEND
COMPOSÉ ORGANIQUE ET DIODE ÉLECTROLUMINESCENTE ORGANIQUE COMPRENANT CELUI-CI

(30) Priority: 05.10.2016 KR 20160128394; 18.09.2017 KR 20170119719
(43) Date of publication of application: 11.04.2018
(60) Divisional application: 24196363.6
(73) Proprietor: SFC Co., Ltd., Cheongju-si, Chungcheongbuk-do 28122 (KR)
(72) Inventor: CHOI, Yeong-Tae, 16954 Yongin-si, Gyeonggi-do (KR); LEE, Se-Jin, 34517 Daejeon (KR); PARK, Seok-Bae, 32737 Geumsan-gun, Chungcheongnam-do (KR); YU, Taejung, 16949 Yongin-si, Gyeonggi-do (KR); YANG, Byung-Sun, 55781 Namwon-si, Jeollabuk-do (KR); LEE, Dajung, 14579 Bucheon-si, Gyeonggi-do (KR)
(74) Representative: Epping - Hermann - Fischer

(56) References cited:
- EP-A1- 3 098 873
- WO-A1-2013/182263
- WO-A1-2017/146474
- US-A1- 2016 351 816

## Description

### BACKGROUND OF THE INVENTION

### 1. Field of the invention

The present disclosure relates to a novel organic compound and an organic light-emitting diode including the same. More particularly, the present disclosure relates to a novel organic compound that can guarantee the device properties of high efficiency, long lifespan, and low-voltage driving when used in an organic light-emitting diode, and an organic light-emitting diode including the same.

### 2. Description of the Prior Art

Organic light-emitting diodes (OLEDs), based on self-luminescence, enjoy the advantage of having a wide viewing angle and being able to be made thinner and lighter than liquid crystal displays (LCDs). In addition, an OLED display exhibits a very fast response time. Accordingly, OLEDs find applications in the illumination field as well as the full-color display field.

In general, the term "organic light-emitting phenomenon" refers to a phenomenon in which electrical energy is converted to light energy by means of an organic material. An OLED using the organic light-emitting phenomenon has a structure usually comprising an anode, a cathode, and an organic material layer interposed therebetween.

In this regard, the organic material layer may be, for the most part, of a multilayer structure consisting of different materials, for example, a hole injection layer, a hole transport layer, a light-emitting layer, an electron transport layer, and an electron injection layer. In the organic light-emitting diode having such a structure, when a voltage is applied between the two electrodes, a hole injected from the anode migrates to the organic layer while an electron is released from the cathode and moves toward the organic layer. In the luminescent zone, the hole and the electron recombine to produce an exciton. When the exciton returns to the ground state from the excited state, the molecule of the organic layer emits light. Such an organic light-emitting diode is known to have characteristics such as self-luminescence, high luminance, high efficiency, low driving voltage, a wide viewing angle, high contrast, and high-speed response.

Materials used as organic layers in OLEDs may be divided into luminescent materials and charge transport materials, for example, a hole injection material, a hole transport material, an electron injection material, and an electron transport material and, as needed, further into an electron-blocking material or a hole-blocking material.

With regard to related arts pertaining to hole transport layers, reference may be made to Korean Patent No. 10-1074193 (issued October 14, 2011), which describes an organic light-emitting diode using a carbazole structure fused with at least one benzene ring in a hole transport layer, and Korean Patent No. 10-1455156 (issued October 27, 2014), which describes an organic light-emitting diode in which the HOMO energy level of an auxiliary light-emitting layer is set between those of a hole transport layer and a light-emitting layer.

In spite of enormous effort for fabricating organic light-emitting diodes having more effective luminous properties, there is still continued need to develop novel organic light-emitting materials suitable for use in hole injection layers and hole transport layers of organic light-emitting diodes that can operate at lower voltage with higher light emission efficiency and longer lifespan, compared to those developed based on conventional technology.

Each of documents WO 2013/182263 A1 and EP 3 098 873 A1 (which is a document according to Art. 54(3) EPC) relates to organic compounds for use as components in OLEDs. Document WO 2017/146474 A1 discloses heterocyclic compounds and an organic light emitting diode containing the same.

### [Related Art Document]

Korean Patent No. 10-1074193 (issued October 14, 2011)
Korean Patent No. 10-1455156 (issued October 27, 2014)

### SUMMARY OF THE INVENTION

Accordingly, a purpose of the present disclosure is to provide an organic compound having a specific structure, which is available for use in a hole transport layer of an OLED to guarantee a high efficiency, a low voltage, and a long lifespan for the OLED.

Another purpose of the present disclosure is to provide a novel OLED in which a hole transport layer contains the organic compound.

In order to accomplish the purposes thereof, the present disclosure provides an organic compound according to claim 1 and an organic light-emitting diode according to claim 8. Preferred embodiments are set forth in the subclaims.

### BRIEF DESCRIPTION OF THE DRAWINGS

The above and other aspects, features and advantages of the present invention will be more apparent from the following detailed description taken in conjunction with the accompanying drawing, in which:

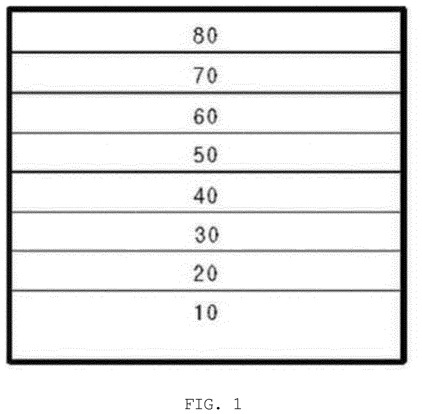

FIG. 1 is a schematic diagram of an OLED according to one embodiment of the present disclosure.

### DETAILED DESCRIPTION OF THE EXEMPLARY EMBODIMENTS

Below, a detailed description is given of the present disclosure.

An organic compound according to the present disclosure has the structure according to claim 1. Preferred embodiments are set forth in claims 2 to 7.

In accordance with another aspect thereof, the present disclosure addresses an organic light-emitting diode, comprising a first electrode; a second electrode facing the first electrode; and an organic layer interposed between the first electrode and the second electrode, wherein the organic layer includes at least one of the organic compounds according to the present disclosure.

As used herein, the expression "(the organic layer)... includes at least one of the organic compounds" is construed to mean that the organic layer may include one or two or more different organic compounds that fall within the scope of the present disclosure.

In this regard, the organic light-emitting diode of the present disclosure may further comprise at least one of a hole injection layer, a hole transport layer, a functional layer capable of both hole injection and hole transport, a light-emitting layer, an electron transport layer, and an electron injection layer.

The organic layer including the organic compound can be used in at least one of a hole injection layer, a hole transport layer, a functional layer capable of both hole injection and hole transport, a light-emitting layer, an electron transport layer, and an electron injection layer, and particularly, the organic compound may be recruited in a hole transport layer.

As a material for a hole transport layer in the organic light-emitting diode according to the present disclosure, the organic compound represented by any one of Chemical Formulas A to E of the present disclosure may be used. Other compounds in addition to the organic compound represented by Chemical Formulas A to E may be further contained in the hole transport layer. Compounds that are typically accepted in the art may be used in the hole transport layer.

For use as a material in a hole transport layer, an electron donating molecule having a low ionization potential is used. Predominantly, diamine, triamine or tetraamine derivatives having a triphenylamine skeleton are employed, as exemplified by N,N'-bis(3-methylphenyl)-N,N'-diphenyl-[1,1-biphenyl]-4,4'-diamine (TPD) and N,N'-di(naphthalen-1-yl)-N,N'-diphenylbenzidine (a-NPD).

A hole injection layer (HIL) may be further deposited beneath the hole transport layer. The organic compound represented by any one of Chemical Formulas A to E may also be used as a material for the hole injection layer. Otherwise, a compound that is typically used in the art may be employed in the hole injection layer.

Examples of such typical compounds for use in a hole injection layer include HATCN (Hexaazatriphenylenehexacarbonitrile), and the starburst amines TCTA (4,4',4"-tri(N-carbazolyl)triphenyl-amine) and m-MTDATA (4,4',4"-tris-(3-methylphenylphenyl amino)triphenylamine).

In addition, a light-emitting layer in the organic light-emitting diode of the present disclosure may include a host and a dopant. For this, the host in the light-emitting layer may include, but is not limited to, at least one of the compounds represented by the following Chemical Formula K: 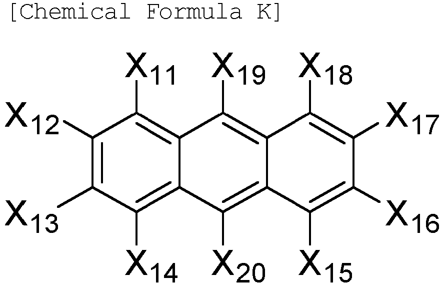 wherein,
X11 to X20, which may be the same or different, are each independently selected from the group consisting of a hydrogen atom, a deuterium atom, a substituted or unsubstituted alkyl of 1 to 30 carbon atoms, a substituted or unsubstituted alkenyl of 2 to 30 carbon atoms, a substituted or unsubstituted cycloalkyl of 3 to 30 carbon atoms, a substituted or unsubstituted cycloalkenyl of 5 to 30 carbon atoms, a substituted or unsubstituted alkoxy of 1 to 30 carbon atoms, a substituted or unsubstituted aryloxy of 6 to 30 carbon atoms, a substituted or unsubstituted alkylthioxy of 1 to 30 carbon atoms, a substituted or unsubstituted arylthioxy of 6 to 30 carbon atoms, a substituted or unsubstituted alkylamine of 1 to 30 carbon atoms, a substituted or unsubstituted arylamine of 6 to 30 carbon atoms, a substituted or unsubstituted aryl of 6 to 50 carbon atoms, a substituted or unsubstituted heteroaryl of 2 to 50 carbon atoms bearing O, N, or S as a heteroatom, a substituted or unsubstituted silicone, a substituted or unsubstituted boron, a substituted or unsubstituted silane, a carbonyl, a phosphoryl, an amino, a nitrile, a hydroxy, a nitro, a halogen, an amide, and an ester, with a proviso that adjacent radicals may form an aliphatic, an aromatic, an aliphatic hetero, or an aromatic hetero fused ring.

Meanwhile, a dopant used in a light-emitting layer of the organic light-emitting diode according to the present disclosure may be a compound represented by the following Chemical Formula 1 or 2. In this regard, the light-emitting layer may further contain various dopant materials known in the art. 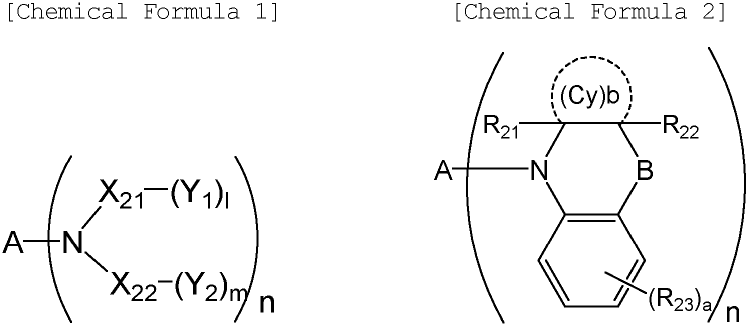 wherein,
A may be any one selected from among a substituted or unsubstituted aryl of 6 to 50 carbon atoms, a substituted or unsubstituted heteroaryl of 2 to 50 carbon atoms bearing O, N, or S as a heteroatom, a substituted or unsubstituted arylene of 6 to 60 carbon atoms, and a substituted or unsubstituted heteroarylene of 3 to 50 carbon atoms bearing O, N, or S as a heteroatom.

In greater detail, A may be a substituted or unsubstituted arylene of 6 to 60 carbon atoms, or a single bond, and particularly any one selected from among anthracene, pyrene, phenanthrene, indenophenanthrene, chrysene, naphthacene, pycene, triphenylene, perylene, and pentacene, and more particularly a substituent represented by the following Chemical Formulas A1 to A10:

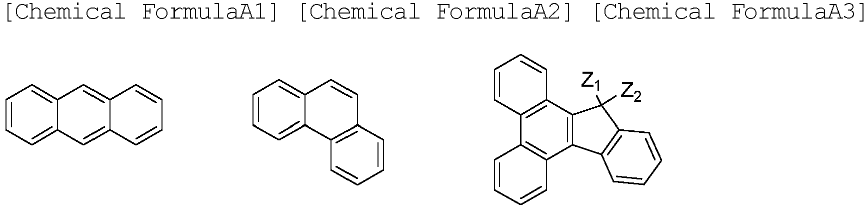

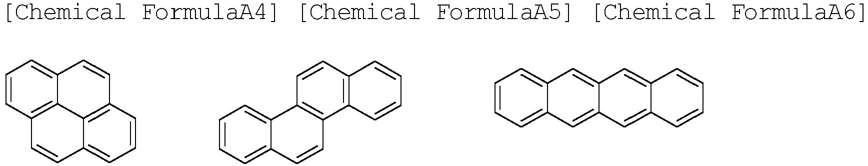

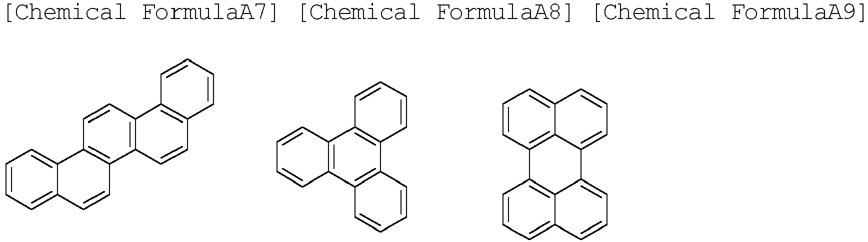

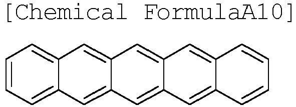

In Chemical Formula A3, Z1 and Z2 may be the same or different and are each independently selected from the group consisting of a hydrogen atom, a deuterium atom,
a substituted or unsubstituted alkyl of 1 to 60 carbon atoms, a substituted or unsubstituted alkenyl of 2 to 60 carbon atoms, a substituted or unsubstituted alkynyl of 2 to 60 carbon atoms, a substituted or unsubstituted alkoxy of 1 to 60 carbon atoms, a substituted or unsubstituted alkylthio of 1 to 60 carbon atoms, a substituted or unsubstituted cycloalkyl of 3 to 60 carbon atoms, a substituted or unsubstituted aryl of 6 to 60 carbon atoms, a substituted or unsubstituted aryloxy of 5 to 60 carbon atoms, a substituted or unsubstituted arylthio of 5 to 60 carbon atoms, a substituted or unsubstituted heteroaryl of 2 to 60 carbon atoms, a substituted or unsubstituted (alkyl)amino of 1 to 60 carbon atoms, a di(substituted or unsubstituted alkyl)amino of 1 to 60 carbon atoms or a (substituted or unsubstituted aryl)amino of 6 to 60 carbon atoms, and a di(substituted or unsubstituted aryl)amino of 6 to 60 carbon atoms, with the proviso that Z1 and Z2 may the same or different and each form a fused ring with an adjacent radical.

In Chemical Formula 1,
X21 and X22 may each be independently a substituted or unsubstituted arylene of 6 to 30 carbon atoms or a single bond, with the proviso that X21 and X22 may bond to each other,
Y1 and Y2 may be the same or different and are each independently selected from the group consisting of a substituted or unsubstituted aryl of 6 to 24 carbon atoms, a substituted or unsubstituted heteroaryl of 2 to 24 carbon atoms, a substituted or unsubstituted alkyl of 1 to 24 carbon atoms, a substituted or unsubstituted heteroalkyl of 1 to 24 carbon atoms, a substituted or unsubstituted cycloalkyl of 3 to 24 carbon atoms, a substituted or unsubstituted alkoxy of 1 to 24 carbon atoms, a cyano, a halogen, a substituted or unsubstituted aryloxy of 6 to 24 carbon atoms, a substituted or unsubstituted alkylsilyl of 1 to 40 carbon atoms, a substituted or unsubstituted arylsilyl of 6 to 30 carbon atoms, a germanium, a phosphorus, a boron, a deuterium, and a hydrogen, with the proviso that Y1 and Y2 may each form with an aliphatic, aromatic, heteroaliphatic, or heteroaromatic fused ring with an adjacent radical,
l and m are each an integer of 1 to 20, and
n is an integer of 1 to 4.
In Chemical Formula 2,
Cy is a substituted or unsubstituted cycloalkyl of 3 to 8 carbon atoms and b is an integer of 1 to 4, with the proviso that when b is an integer of 2 or greater, the corresponding cycloalkanes may be the same or different and may each be in a fused form having a deuterium or an alkyl as a substituent;
B is a single bond or -[C(R25)(R26)]p- wherein p is an integer of 1 to 3, with the proviso that when p is 2 or greater, the corresponding two or more R25's are the same or different and the corresponding two or more R26's are the same or different;
R21, R22, R23, R25, and R26 may each be independently selected from among a hydrogen atom, a deuterium atom, a halogen atom, a hydroxyl, a cyano, a nitro, an amino, an amidino, a hydrazine, a hydrazone, a carboxyl or a salt thereof, a sulfonic acid or a salt thereof, a phosphoric acid or a salt thereof, a substituted or unsubstituted alkyl of 1 to 60 carbon atoms, a substituted or unsubstituted alkenyl of 2 to 60 carbon atoms, a substituted or unsubstituted alkynyl of 2 to 60 carbon atoms, a substituted or unsubstituted alkoxy of 1 to 60 carbon atoms, a substituted or unsubstituted alkylthio of 1 to 60 carbon atoms, a substituted or unsubstituted cycloalkyl of 3 to 60 carbon atoms, a substituted or unsubstituted aryl of 6 to 60 carbon atoms, a substituted or unsubstituted aryloxy of 5 to 60 carbon atoms, a substituted or unsubstituted arylthio of 5 to 60 carbon atoms, a substituted or unsubstituted heteroaryl of 2 to 60 carbon atoms, a substituted or unsubstituted (alkyl)amino of 1 to 60 carbon atoms, a di(substituted or unsubstituted alkyl)amino of 1 to 60 carbon atoms or a (substituted or unsubstituted aryl)amino of 6 to 60 carbon atoms, a di(substituted or unsubstituted aryl)amino of 6 to 60 carbon atoms, a substituted or unsubstituted alkylsilyl of 1 to 40 carbon atoms, a substituted or unsubstituted arylsilyl of 6 to 30 carbon atoms, a germanium, a phosphorus, and a boron,
a is an integer of 1 to 4, with the proviso that when a is 2 or greater, the corresponding plural R23's may be the same or different and may each be in a fused form, and
n is an integer of 1 to 4.

The amine radical of Chemical Formulas 1 and 2, which is linked to A, may be represented by any one selected from among, but not limited to, the following Substituents 1 to 52: 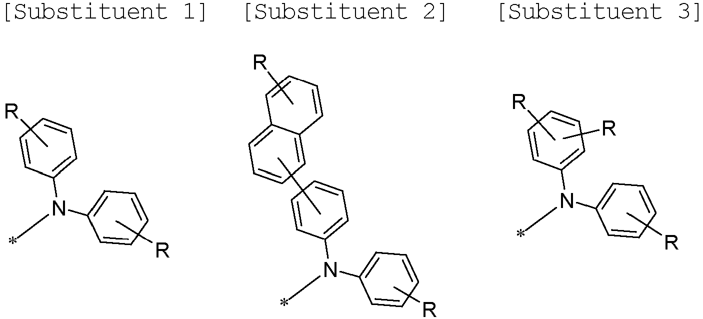 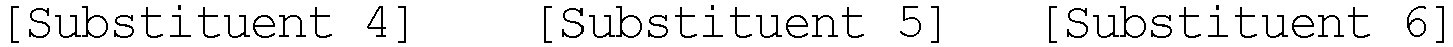 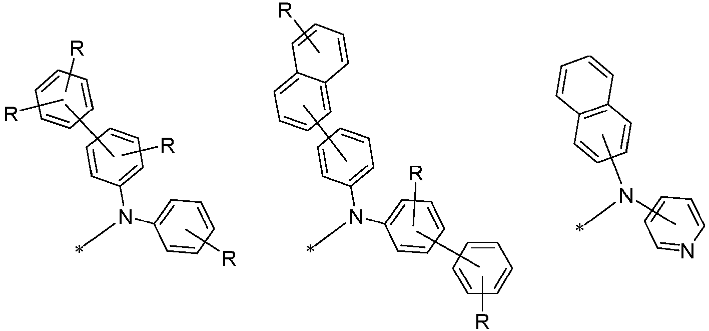 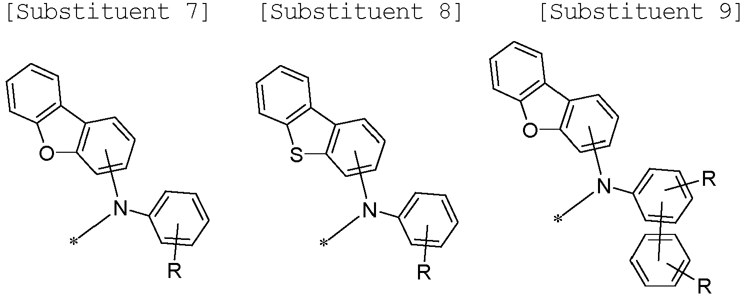 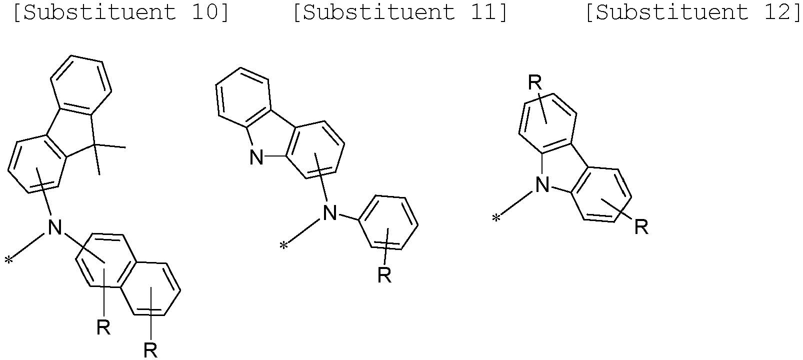 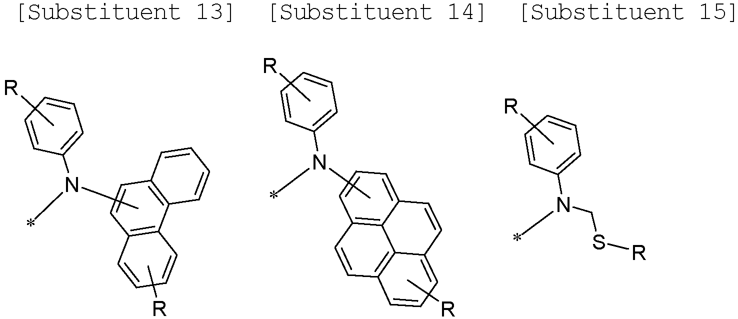 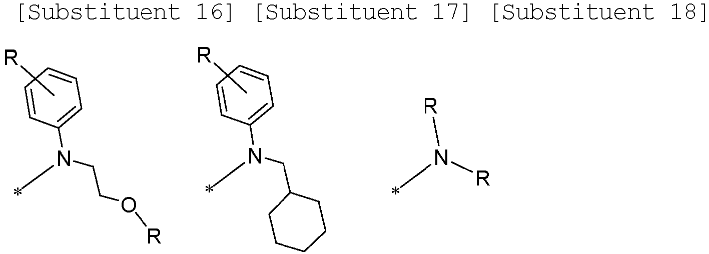 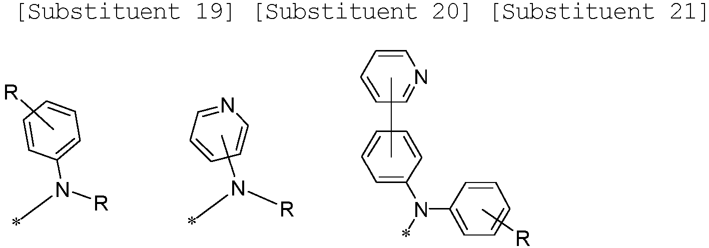 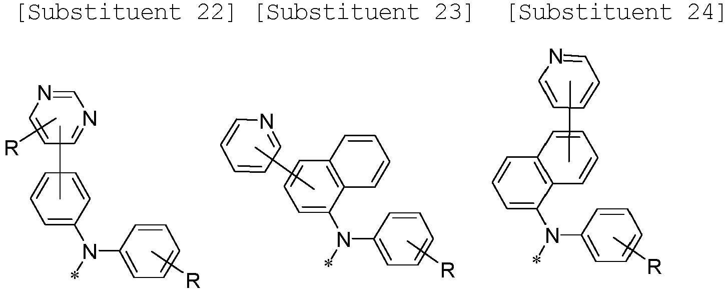 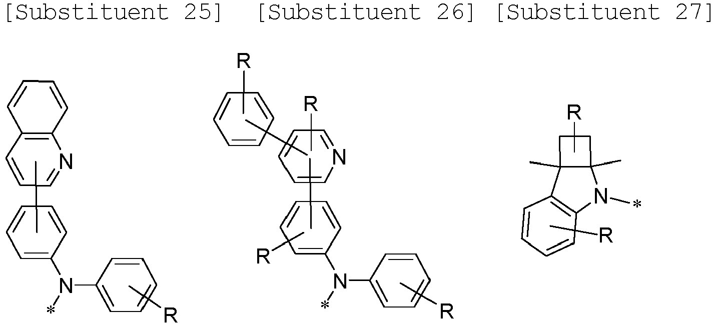 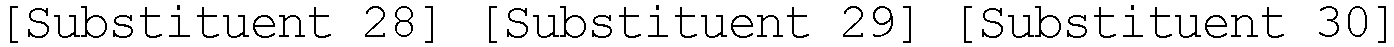 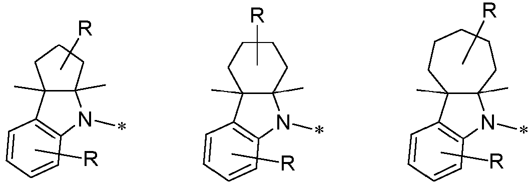 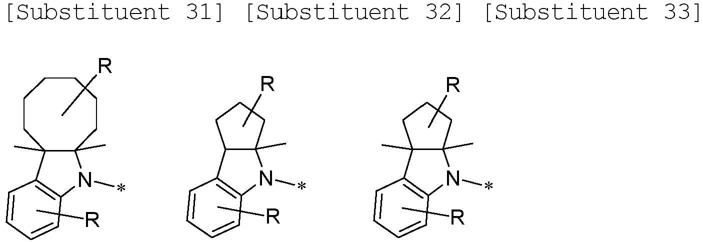 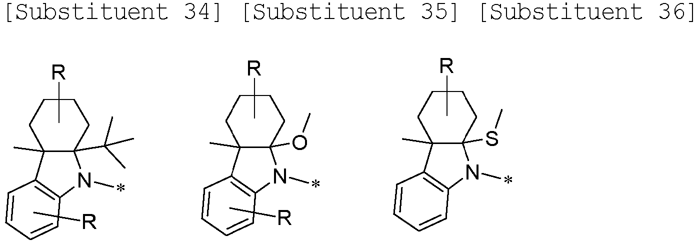 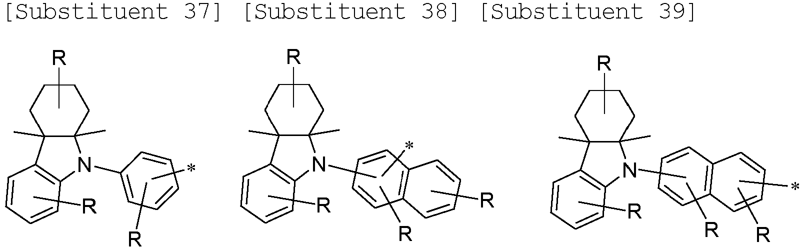 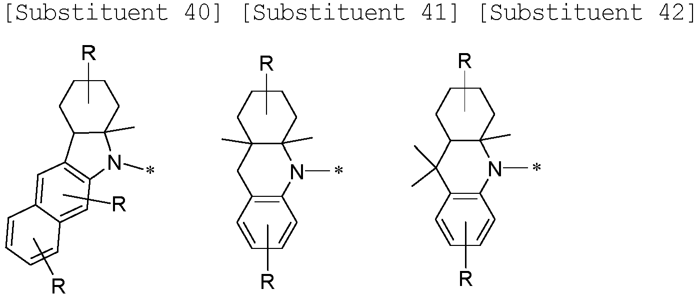 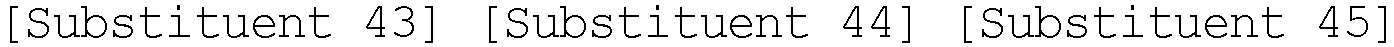 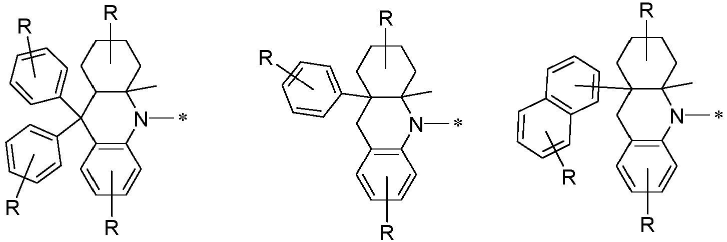 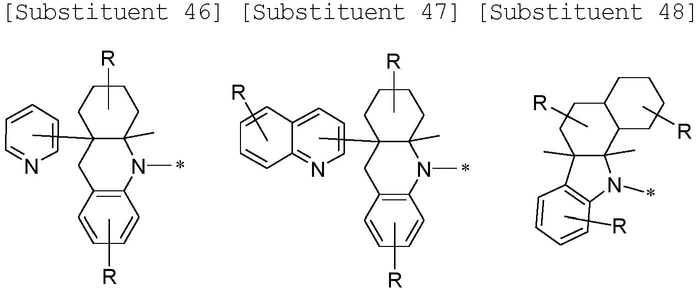 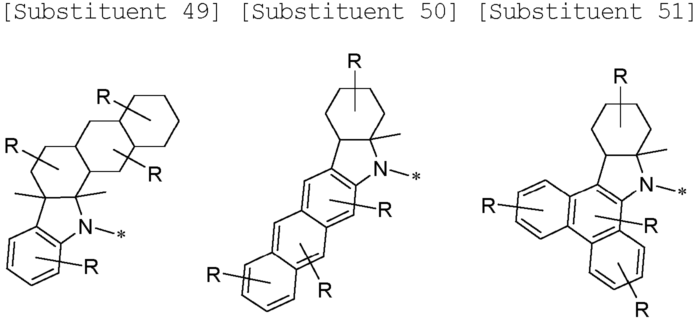 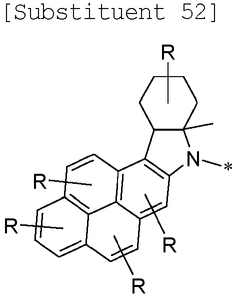 wherein R's, which may be the same or different, are each independently selected from among a hydrogen atom, a deuterium atom, a halogen atom, a hydroxyl, a cyano, a nitro, an amino, an amidino, a hydrazine, a hydrazone, a carboxyl or a salt thereof, a sulfonic acid or a salt thereof, a phosphoric acid or a salt thereof, a substituted or unsubstituted alkyl of 1 to 60 carbon atoms, a substituted or unsubstituted alkenyl of 2 to 60 carbon atoms, a substituted or unsubstituted alkynyl of 2 to 60 carbon atoms, a substituted or unsubstituted alkoxy of 1 to 60 carbon atoms, a substituted or unsubstituted alkylthio of 1 to 60 carbon atoms, a substituted or unsubstituted cycloalkyl of 3 to 60 carbon atoms, a substituted or unsubstituted aryl of 6 to 60 carbon atoms, a substituted or unsubstituted aryloxy of 5 to 60 carbon atoms, a substituted or unsubstituted arylthio of 5 to 60 carbon atoms, a substituted or unsubstituted heteroaryl of 2 to 60 carbon atoms, a substituted or unsubstituted (alkyl)amino of 1 to 60 carbon atoms, a di(substituted or unsubstituted alkyl)amino of 1 to 60 carbon atoms or a (substituted or unsubstituted aryl)amino of 6 to 60 carbon atoms, a di(substituted or unsubstituted aryl)amino of 6 to 60 carbon atoms, a substituted or unsubstituted alkylsilyl of 1 to 40 carbon atoms, a substituted or unsubstituted arylsilyl of 6 to 30 carbon atoms, may each have 1 to 12 substituents, and may each form a fused ring with an adjacent radical.

The light-emitting layer may contain various host and dopant materials in addition to the aforementioned dopant and host materials.

When the light-emitting layer contains a host and a dopant, the content of the dopant in the light-emitting layer may range from about 0.01 to 20 parts by weight based on 100 parts by weight of the host, but is not limited thereto.

Meanwhile, examples of the material typically used in an electron transport layer include quinoline derivatives, particularly tris(8-quinolinolate)aluminum (Alq3), TAZ, BAlq, beryllium bis(benzoquinolin-10-oate: Bebq2), BCP, compound 201, compound 202, and the oxadiazole derivatives PBD, BMD, and BND, but are not limited thereto.

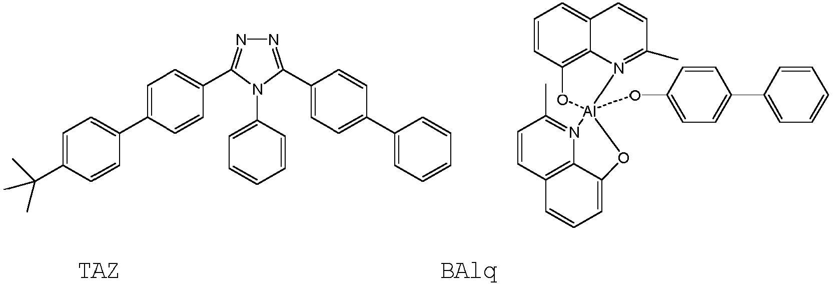

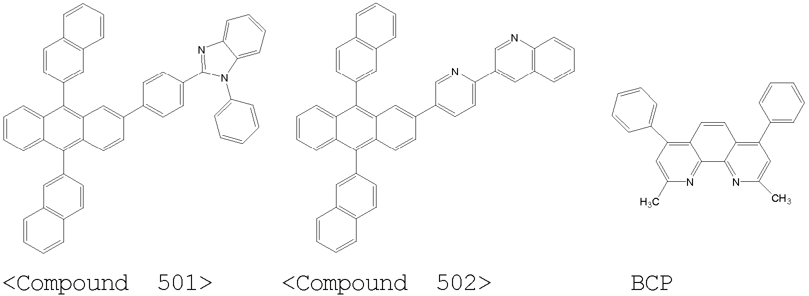

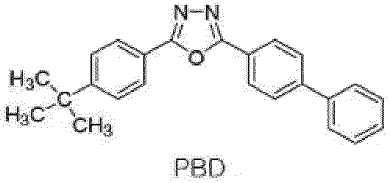

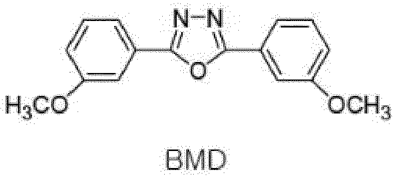

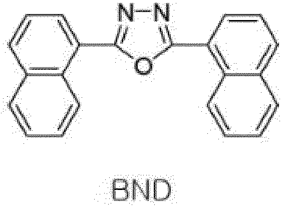

In addition, the organic metal compound represented by Chemical Formula F may be used, either alone or in combination with the aforementioned material, as a compound for an electron transport layer in the present disclosure: 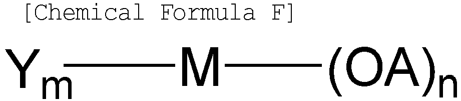 wherein,
Y is a ligand that contains two moieties respectively responsible for forming a single bond through a direct bond to M and for forming a coordinate bond with M, each moiety being selected from among C, N, O and S, and which is chelated by the single bond and the coordinate bond;
M is an alkali metal, an alkaline earth metal, an aluminum (Al) atom, or a boron (B) atom, with the proviso that:
   when M is an alkali metal, m=1 and n=0
   when M is an alkaline earth metal, m=1 and n=1, or m=2 and n=0, or
   when M is aluminum or a boron, m is an integer of 1 to 3 and n is an integer of 0 to 2, satisfying the relationship m +n=3; and
   OA is a monodentate ligand capable of forming a single bond or a coordinate bond with M,
   wherein O is oxygen, and A is selected from among a substituted or unsubstituted alkyl of 1 to 30 carbon atoms, a substituted or unsubstituted aryl of 5 to 50 carbon atoms, a substituted or unsubstituted alkenyl of 2 to 30 carbon atoms, a substituted or unsubstituted alkynyl of 2 to 20 carbon atoms, a substituted or unsubstituted cycloalkyl of 3 to 30 carbon atoms, a substituted or unsubstituted cycloalkenyl of 5 to 30 carbon atoms, and a substituted or unsubstituted heteroaryl of 2 to 50 carbon atoms bearing O, N, or S as a heteroatom.

An electron injection layer (EIL) that functions to facilitate electron injection from the cathode, thus improving the power efficiency of the diode, may be further deposited on the electron transport layer. So long as it is conventionally used in the art, any material can be available for the electron injection layer without particular limitations. Examples include LiF, NaCl, CsF, Li2O, and BaO.

Deposition conditions for the electron injection layer may vary, depending on compounds used, but may be generally selected from condition scopes that are almost the same as for the formation of hole injection layers.

The electron injection layer may range in thickness from about 1 Å to about 100 Å, and particularly from about 3 Å to about 90 Å. Given the thickness range for the electron injection layer, the diode can exhibit satisfactory electron injection properties without actually elevating a driving voltage.

Here, the cathode may be made of lithium (Li), magnesium (Mg), aluminum (Al), aluminum-lithium (Al-Li), calcium (Ca), magnesium-indium (Mg-In), or magnesium-silver (Mg-Ag). For a top-emitting OLED, a transparent cathode made of ITO or IZO may be employed.

Further, one or more layers selected from among a hole injecting layer, a hole transport layer, a functional layer capable of both hole injection and hole transport, a light-emitting layer, an electron transport layer, and an electron injection layer may be deposited using a deposition process or a solution process.

Here, the deposition process is a process by which a material is vaporized and deposited in a vacuum or at a low pressure to form a layer, and the solution process is a method in which a material is dissolved in a solvent and applied for the formation of a thin film by means of inkjet printing, roll-to-roll coating, screen printing, spray coating, dip coating, spin coating, etc.

Also, the organic light-emitting diode of the present disclosure may be applied to a device selected from among flat display devices, flexible display devices, monochrome or grayscale flat illumination devices, and monochrome or grayscale flexible illumination devices.

Below, the organic light-emitting diode of the present disclosure is explained with reference to FIG. 1.

FIG. 1 is a schematic cross-sectional view of the structure of an organic light-emitting diode according to some embodiments of the present disclosure. The organic light-emitting diode includes an anode 20, a hole transport layer 40, an organic light-emitting layer 50, an electron transport layer 60, and a cathode 80, and optionally a hole injection layer 30 or an electron injection layer 70. In addition, one or two intermediate layers may be further formed in the organic light-emitting diode. A hole barrier layer or an electron barrier layer may be also further established.

Reference is made to FIG. 1 with regard to the organic light-emitting diode of the present disclosure and the fabrication thereof. First, a substrate 10 is coated with an anode electrode material to form an anode 20. So long as it is used in a typical organic EL device, any substrate may be used as the substrate 10. Preferable is an organic substrate or transparent plastic substrate that exhibits excellent transparency, surface smoothness, ease of handling, and waterproofness. As the anode electrode material, indium tin oxide (ITO), indium zinc oxide (IZO), tin oxide (SnO2), or zinc oxide (ZnO), which are transparent and superior in terms of conductivity, may be used.

A hole injection layer material is applied on the anode electrode 20 by thermal deposition in a vacuum or by spin coating to form a hole injection layer 30. Subsequently, using thermal deposition in a vacuum or spin coating, a hole transport layer material is applied to the hole injection layer 30 to form a hole transport layer 40.

Then, an organic light-emitting layer 50 is deposited on the hole transport layer 40, optionally followed by the formation of a hole barrier layer (not shown) on the organic light-emitting layer 50 by deposition in a vacuum or by spin coating. When holes traverse the organic light-emitting layer and are introduced into the cathode, the efficiency and lifespan of the diode are deteriorated. Formed of a material with a low HOMO (Highest Occupied Molecular Orbital) level, the hole barrier layer serves to prevent the introduction of holes into the cathode. Any material that has a higher ionization potential than the light-emitting compound and which is also able to carry electrons may be used for the hole barrier layer without limitation. Representative among hole barrier materials are BAlq, BCP, and TPBI.

Using a vacuum deposition method or a spin-coating method, an electron transport layer 60 may be deposited on the hole barrier layer and may then be overlaid with an electron injection layer 70. A cathode metal is deposited on the electron injection layer 70 by thermal deposition in a vacuum to form a cathode 80, thus obtaining an organic EL diode. Here, the cathode may be made of lithium (Li), magnesium (Mg), aluminum (Al), aluminum-lithium (Al-Li), calcium (Ca), magnesium-indium (Mg-In), or magnesium-silver (Mg-Ag). For a top-emitting OLED, a transparent cathode made of ITO or IZO may be employed.

A better understanding of the present disclosure may be obtained through the following examples which are set forth to illustrate, but are not to be construed as limiting the present disclosure.

### SYNTHESIS EXAMPLE 1: Synthesis of [Compound 1]

### Synthesis Example 1-1: Synthesis of [Intermediate 1-a]

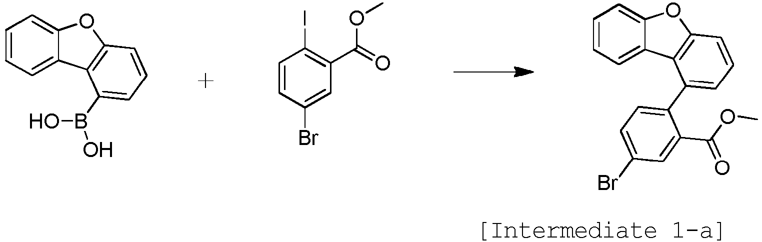

Dibenzofuran-1-boronic acid (25 g, 118 mmol), methyl-5-bromo-2-iodobenzoate (40.5 g, 118 mmol), tetrakis(triphenylphosphine)palladium (2.7 g, 2.3 mmol), potassium carbonate (33 g, 237 mmol), toluene (200 ml), 1,4-dioxane (200 ml), and water (100 ml) were placed under a nitrogen atmosphere in a round-bottom flask and fluxed for 12 hours. After completion of the reaction, the reaction mixture was layer separated, and the organic layer was concentrated at a reduced pressure. Column chromatographic separation yielded Intermediate 1-a (33.5 g): yield 74%

### Synthesis Example 1-2: Synthesis of [Intermediate 1-b]

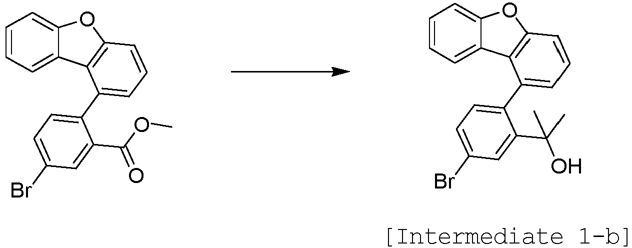

In a round-bottom flask, Intermediate 1-a (33.5 g, 110 mmol) was added to tetrahydrofuran (150 ml) and cooled to -10°C before slow addition of drops of 3 M methyl magnesium bromide (85 ml, 254 mmol) thereto. After the temperature was elevated to 40°C, stirring was performed for 4 hours. Then, the temperature was reduced to -10°C and drops of 2 N HCl (70 ml) were slowly added. Subsequently, an aqueous ammonium chloride solution (70 ml) was added, followed by elevation to room temperature. After completion of the reaction, the reaction mixture was washed with water, concentrated in a vacuum, and separated by column chromatography to afford [Intermediate 1-b] (27 g): yield 80%

### Synthesis Example 1-3: Synthesis of [Intermediate 1-c]

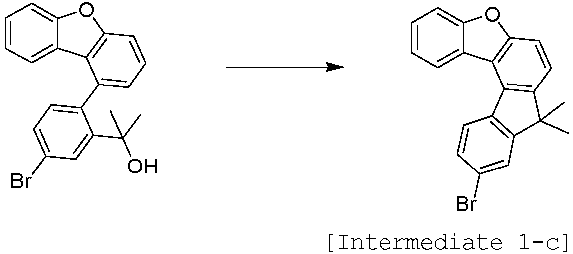

In a round-bottom flask, Intermediate 1-b (27 g, 89.2 mmol) and phosphoric acid (70 ml) were stirred together at room temperature for 12 hours under a nitrogen atmosphere. After completion of the reaction, the reaction mixture was extracted, concentrated, and separated by column chromatography to afford Intermediate 1-c (17.6 g): yield 70%

### Synthesis Example 1-4 (Reference): Synthesis of [Compound 1]

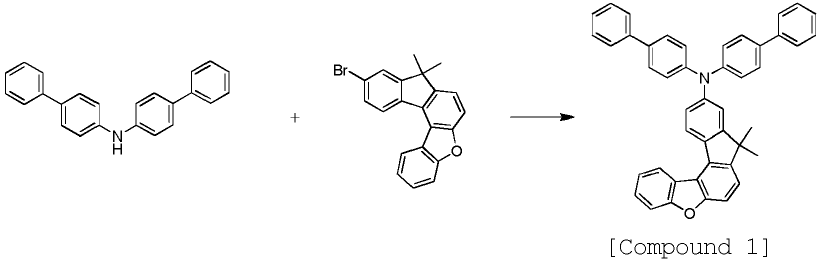

In a round-bottom flask, Intermediate 1-c (10 g, 27.5 mmol), bis(4-biphenyl)amine (8.8 g, 27.5 mmol), bis(dibenzylideneacetone)dipalladium (0.5 g, 0.6 mmol), BINAP (0.7 g, 1.1 mmol), sodium tert-butoxide (6.6 g, 68.8 mmol), and toluene (100 ml) were fluxed for 12 hours under a nitrogen atmosphere. After completion of the reaction, the organic layer was concentrated in a vacuum. Column chromatographic separation afforded Compound 1 (11.8 g): yield 72%
MS (MALDI-TOF): m/z 603.26 [M]+

### SYNTHESIS EXAMPLE 2 (Reference): Synthesis of [Compound 3]

### Synthesis Example 2-1: Synthesis of [Compound 3]

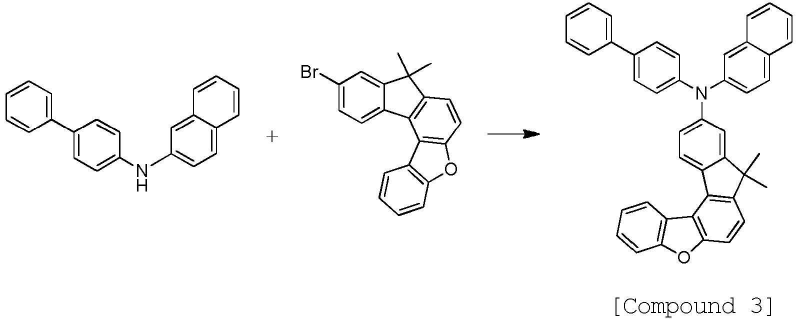

The same procedure as in Synthesis Example 1-4 was carried out, with the exception of using N-(naphthalen-2-yl)biphenyl-4-amine instead of bis(4-biphenyl)amine, to afford Compound 3: yield 67%
MS (MALDI-TOF): m/z 577.24 [M]+

### SYNTHEIS EXAMPLE 3 (Reference): Synthesis of [Compound 67]

### Synthesis Example 3-1: Synthesis of [Intermediate 3-a]

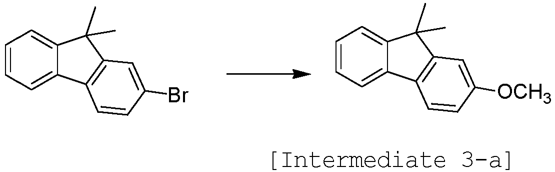

In a round-bottom flask, 2-bromo-9,9-dimethylfluorene (50 g, 183 mmol), an aqueous sodium methoxide (59.3 g, 1098 mmol), copper iodide (10.4 g, 54.9 mmol), and methanol (200 ml) were fluxed for 12 hours under a nitrogen atmosphere. After completion of the reaction, the organic layer was concentrated in a vacuum. Column chromatographic separation afforded Intermediate 3-a (33.2 g): yield 81%

### Synthesis Example 3-2: Synthesis of [Intermediate 3-b]

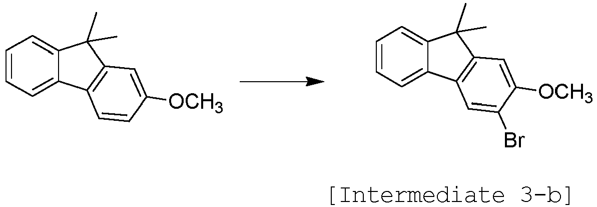

In a round-bottom flask, Intermediate 3-a (30 g, 133 mmol), N-bromosuccinimide (23.8 g, 133 mmol), and dimethylformamide (600 ml) were stirred together at 50°C for 12 hours under a nitrogen atmosphere. After completion of the reaction, the organic layer was concentrated in a vacuum. Column chromatographic separation afforded Intermediate 3-b (28 g): yield 70%

### Synthesis Example 3-3: Synthesis of [Intermediate 3-c]

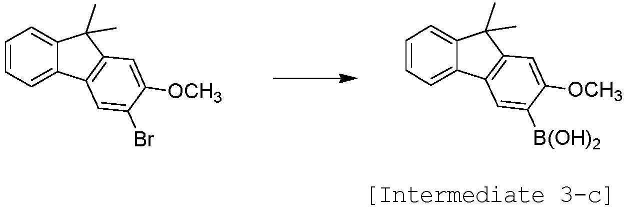

In a round-bottom flask, Intermediate 3-b (49.7 g, 164 mmol) was mixed with tetrahydrofuran (500 ml) and cooled to -78°C under a nitrogen atmosphere, followed by slowly adding drops of 1.6 M butyl lithium (123 ml, 197 mmol). One hour later, trimethyl borate (22 ml, 214 mmol) was slowly fed into the flask, with the low temperature maintained. Subsequently, the reaction mixture was heated to room temperature and then stirred. After completion of the reaction, the organic layer was concentrated in a vacuum and recrystallized in hexane to afford Intermediate 3-c (35.6 g): yield 81%.

### Synthesis Example 3-4: Synthesis of [Intermediate 3-d]

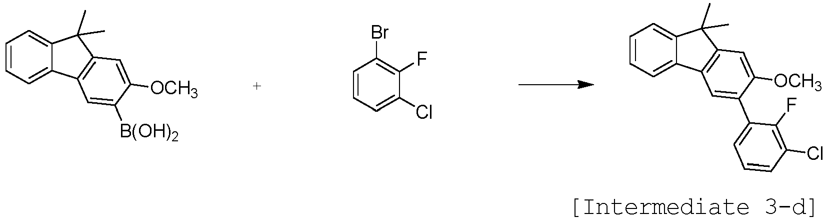

The same procedure as in Synthesis Example 1-1 were carried out, with the exception of using Intermediate 3-c and 1-bromo-3-chloro-2-fluorobenzene instead of dibenzofuran-1-boronic acid and methyl-5-bromo-2-iodobenzoate, respectively, to afford Intermediate 3-d: yield 52%

### Synthesis Example 3-5: Synthesis of [Intermediate 3-e]

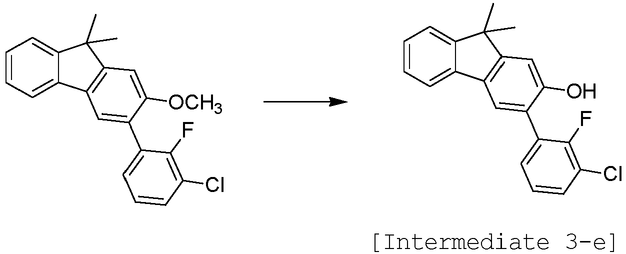

In a round-bottom flask, Intermediate 3-d (30 g, 85 mmol) and dichloromethane (300 ml) were placed under a nitrogen atmosphere, cooled to 0°C, and then slowly added with drops of a dilution of borontribromide (63.9 g, 255 mmol) in dichloromethane (150 ml). The reaction mixture was heated to room temperature and stirred for 6 hours. After completion of the reaction, the organic layer was concentrated in a vacuum. Column chromatographic separation afforded Intermediate 3-e (21.3 g): yield 74%

### Synthesis Example 3-6: Synthesis of [Intermediate 3-f]

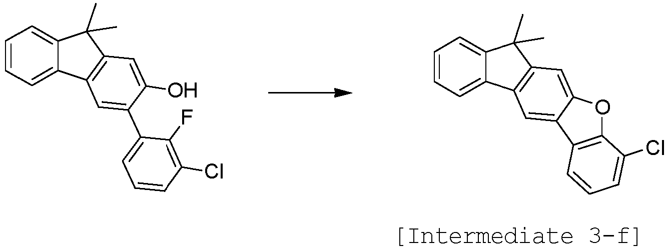

In a round-bottom flask, Intermediate 3-e (20 g, 59 mmol), potassium carbonate (13 g, 94.5 mmol), and 1-methyl-2-pyrrolidinone (200 ml) were stirred together at 150°C for 12 hours under a nitrogen. After completion of the reaction, the organic layer was concentrated in a vacuum. Column chromatographic separation afforded Intermediate 3-f (13.5 g): yield 72%

### Synthesis Example 3-7 (Reference): Synthesis of [Compound 67]

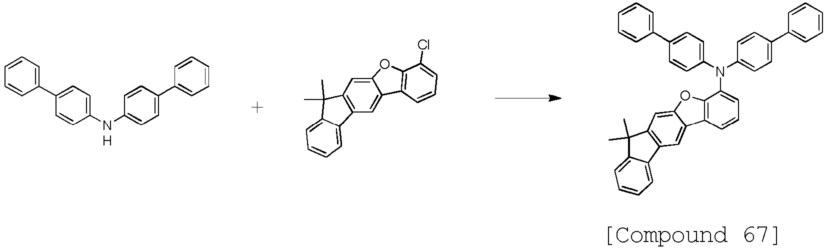

The same procedure as in Synthesis Example 1-4 was carried out, with the exception of using Intermediate 3-f instead of Intermediate 1-c, to afford Compound 67: yield 70%
MS (MALDI-TOF): m/z 603.26 [M]+

### SYNTHESIS EXAMPLE 4 (Reference): Synthesis of [Compound 68]

### Synthesis Example 4-1: Synthesis of [Intermediate 4-a]

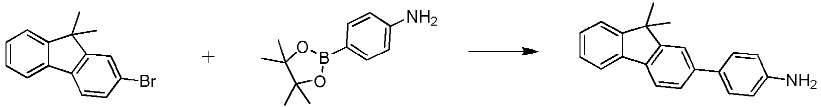

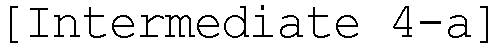

The same procedure as in Synthesis Example 1-1 was carried out, with the exception of using 4-aminophenylboronic acid pinacol ester and 2-bromo-9,9-dimethylfluorene instead of dibenzofuran-1-boronic acid and methyl-5-bromo-2-iodobenzoate, respectively, to afford Intermediate 4-a: yield 72%

### Synthesis Example 4-2: Synthesis of [Intermediate 4-b]

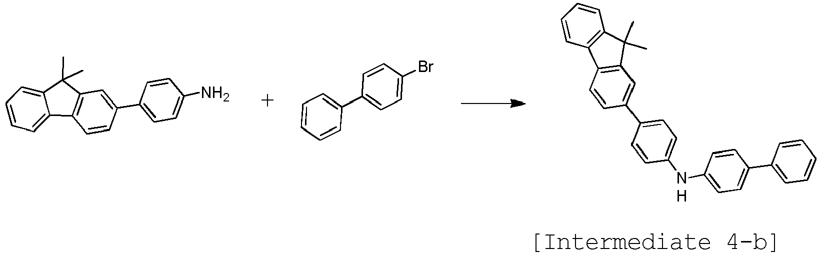

In a 250-ml reactor, Intermediate 4-a (7.0 g, 24.6 mmol), 4-bromobiphenyl (7.0 g, 30 mmol), palladium acetate (0.4 g, 0.3 mmol), sodium tert-butoxide (4.2 g, 43 mmol), tri-tert-butylphosphine (0.1 g, 0.3 mmol), and toluene (100 ml) were stirred together under reflux for 13 hours. After completion of the reaction, the reaction mixture was filtered, and the filtrate was concentrated, and separated by column chromatography to afford Intermediate 4-b (7.0 g): yield 65%

### Synthesis Example 4-3 (Reference): Synthesis of [Compound 68]

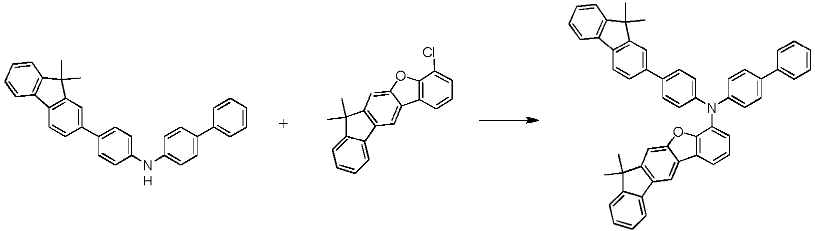

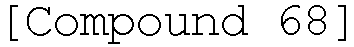

The same procedure as in Synthesis Example 1-4 was carried out, with the exception of using Intermediate 4-b and Intermediate 3-f instead of bis(4-biphenyl)amine and Intermediate 1-c, respectively, to afford [Compound 68]: yield 73%
MS (MALDI-TOF): m/z 719.32 [M]+

### SYNTHESIS EXAMPLE 5 (Reference): Synthesis of [Compound 111]

### Synthesis Example 5-1: Synthesis of [Intermediate 5-a]

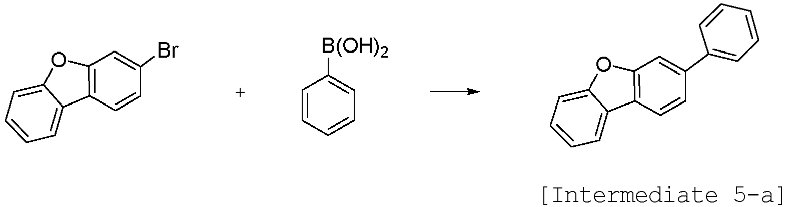

The same procedure as in Synthesis Example 1-1 was carried out, with the exception of using phenylboronic acid and 3-bromodibenzofuran instead of dibenzofuran-1-boronic acid and methyl-5-bromo-2-iodobenzoate, respectively, to afford [Intermediate 5-a]: yield 76%

### Synthesis Example 5-2: Synthesis of [Intermediate 5-b]

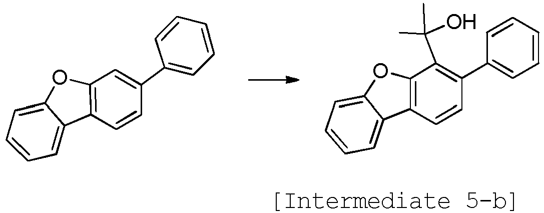

In a round-bottom flask, Intermediate 5-a (50 g, 204 mmol) and tetrahydrofuran (500 ml) were placed, cooled to -78°C, and slowly added with drops of 1.6 M-butyl lithium (128 ml, 204 mmol) before stirring for 1 hours. Drops of acetone (14.3 g, 245 mmol) was slowly added, followed by stirring at room temperature for 6 hours. After completion of the reaction, an aqueous ammonium chloride solution (50 ml) was added to split layers. The organic layer was concentrated in a vacuum and separated by chromatography to afford Intermediate 5-b (38.3 g): yield 62%

### Synthesis Example 5-3: Synthesis of [Intermediate 5-c]

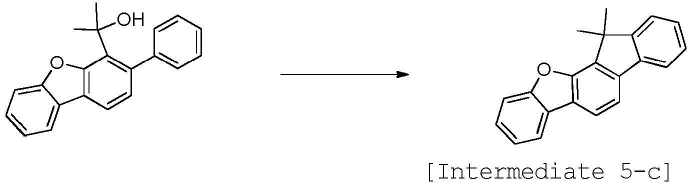

The same procedure as in Synthesis Example 1-3 was carried out, with the exception of using Intermediate 5-b instead of Intermediate 1-b, to afford Intermediate 5-c: yield 69%

### Synthesis Example 5-4: Synthesis of [Intermediate 5-d]

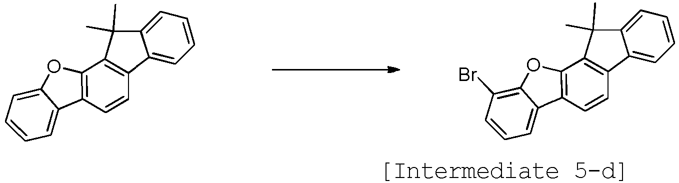

In a round-bottom flask, Intermediate 5-c (14.5 g, 50.9 mmol) was placed and then added with tetrahydrofuran (159 ml) under a nitrogen atmosphere. After the mixture was cooled to -78°C, drops of 1.6 M butyl lithium (35 ml, 56 mmol) were slowly added. After one hour, trimethyl borate (6.3 ml, 61.1 mmol) was slowly added while the low temperature was maintained. Subsequently, stirring was conducted while the temperature was elevated to room temperature. After completion of the reaction, the organic layer was concentrated in a vacuum and then recrystallized in hexane to afford Intermediate 5-d (14.8 g): yield 80%

### Synthesis Example 5-5 (Reference): Synthesis of [Compound 111]

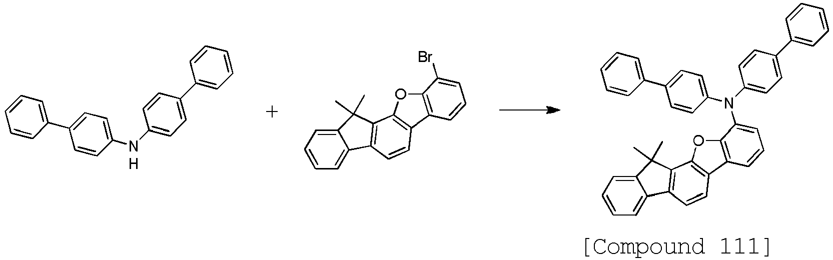

The same procedure as in Synthesis Example 1-4 was carried out, with the exception of using [Intermediate 5-d] instead of [Intermediate 1-c], to afford Compound 111: yield 70%
MS (MALDI-TOF): m/z 603.26 [M]+

### SYNTHESIS EXAMPLE 6 (Reference): Synthesis of [Compound 120]

### Synthesis Example 6-1: Synthesis of [Intermediate 6-a]

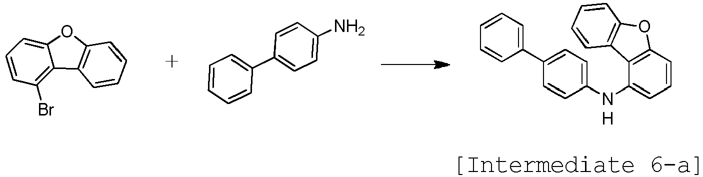

The same procedure as in Synthesis Example 4-2 was carried out, with the exception of using 4-aminobiphenyl and 1-bromodibenzofuran instead of Intermediate 4-a and 4-bromobiphenyl, respectively, to afford Intermediate 6-a: yield 58%

### Synthesis Example 6-2 (Reference): Synthesis of [Compound 120]

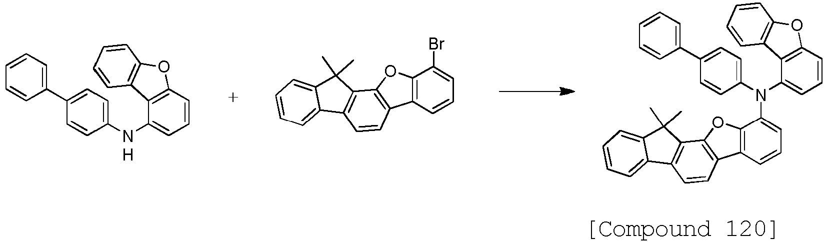

The same procedure as in Synthesis Example 1-4 was carried out, with the exception of using Intermediate 6-a and Intermediate 5-d instead of bis(4-biphenyl)amine and Intermediate 1-c, respectively, to afford Compound 120: yield 70%
MS (MALDI-TOF): m/z 617.24 [M]+

### SYNTHESIS EXAMPLE 7 (Reference): Synthesis of [Compound 136]

### Synthesis Example 7-1: Synthesis of [Intermediate 7-a]

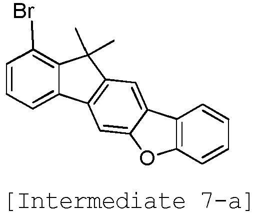

The same procedures as in Synthesis Examples 1-1 to 1-3 were carried out, with the exception of using dibenzofuran-3-boronic acid instead of dibenzofuran-1-boronic acid in Synthesis Example 1-1 and methyl-5-bromo-1-iodobenzoate boronic acid instead of methyl-5-bromo-2-iodobenzoate, to afford Intermediate 7-a: yield 70%

### Synthesis Example 7-2 (Reference): Synthesis of [Compound 136]

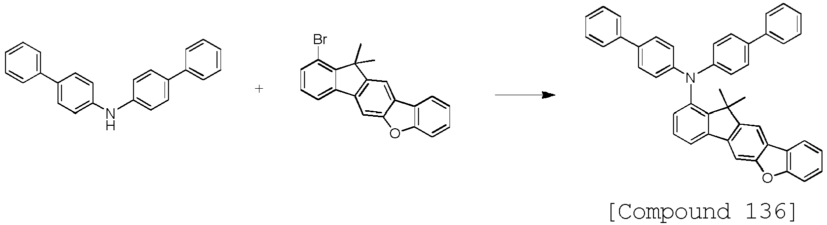

The same procedure as in Synthesis Example 1-4 was carried out, with the exception of using Intermediate 7-a instead of Intermediate 1-c, to afford Compound 136: yield 72%
MS (MALDI-TOF): m/z 603.26 [M]+

### SYNTHESIS EXAMPLE 8 (Reference): Synthesis of [Compound 141]

### Synthesis Example 8-1: Synthesis of [Intermediate 8-a]

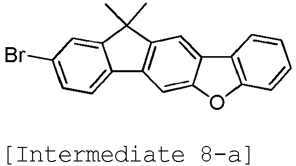

The same procedures as in Synthesis Examples 1-1 to 1-3 were carried out, with the exception of using dibenzofuran-3-boronic acid instead of dibenzofuran-1-boronic acid in Synthesis Example 1-1, to afford Intermediate 8-a: yield 68%

### Synthesis Example 8-2 (Reference): Synthesis of [Compound 141]

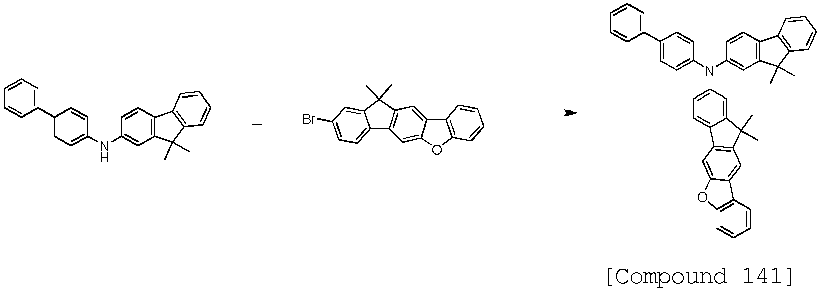

The same procedure as in Synthesis Example 1-4 was carried out, with the exception of using N-(4-biphenyl)-(9,9-dimethylfluoren-2-yl)amine and Intermediate 8-a instead of bis(4-biphenyl)amine and Intermediate 1-c, respectively, to afford Compound 141: yield 75%
MS (MALDI-TOF): m/z 643.29 [M]+

### SYNTHESIS EXAMPLE 9 (Reference): Synthesis of [Compound 181]

### Synthesis Example 9-1: Synthesis of [Intermediate 9-a]

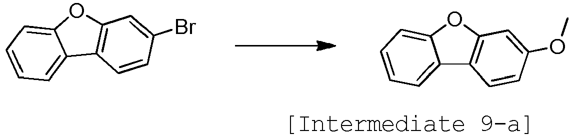

In a round-bottom flask, 3-bromodibenzofuran (50 g, 202 mmol), an aqueous sodium methoxide solution (54.6 g, 1214 mmol), copper iodide (11.5 g, 60.7 mmol), and methanol (200 ml) were fluxed for 12 hours under a nitrogen atmosphere. After completion of the reaction, the organic layer was concentrated in a vacuum. Column chromatographic separation afforded Intermediate 9-a (36 g): yield 90%

### Synthesis Example 9-2: Synthesis of [Intermediate 9-b]

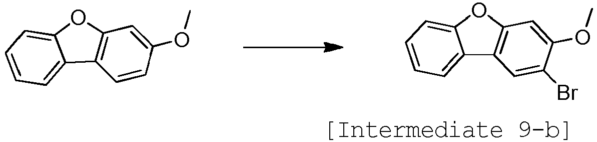

In a round-bottom flask, Intermediate 9-a (36 g, 181 mmol), N-bromosuccinimide (32.3 g, 181 mmol), and dimethyl formamide (700 ml) were stirred together at 50°C for 12 hours under a nitrogen atmosphere. After completion of the reaction, the organic layer was concentrated in a vacuum. Column chromatographic separation afforded Intermediate 9-b (35 g): yield 70%

### Synthesis Example 9-3: Synthesis of [Intermediate 9-c]

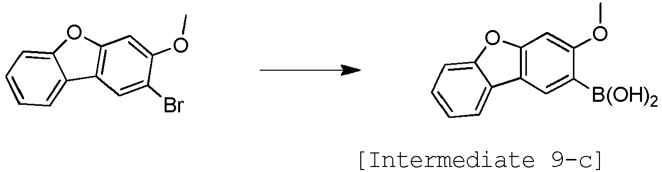

The same procedure as in Synthesis Example 3-3 was carried out, with the exception of using Intermediate 9-b instead of Intermediate 3-b, to afford Intermediate 9-c: yield 78%

### Synthesis Example 9-4: Synthesis of [Intermediate 9-d]

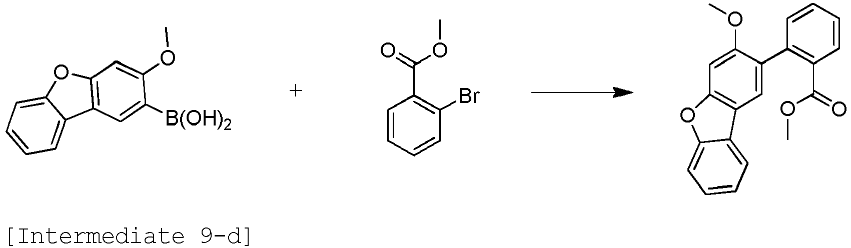

The same procedure as in Synthesis Example 1-1 was carried out, with the exception of using Intermediate 9-c and methyl-2-bromobenzoate instead of dibenzofuran-1-boronic acid and methyl-5-bromo-2-iodobenzoate, respectively, to afford Intermediate 9-d: yield 64%

### Synthesis Example 9-5: Synthesis of [Intermediate 9-e]

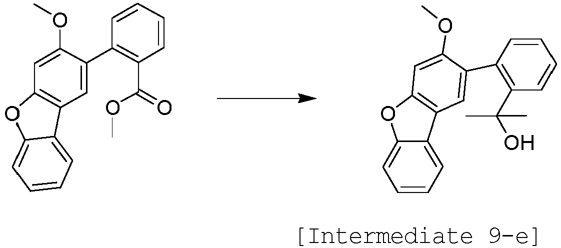

The same procedure as in Synthesis Example 1-2 was carried out, with the exception of using Intermediate 9-d instead of Intermediate 1-a, to afford Intermediate 9-e: yield 77%

### Synthesis Example 9-6: Synthesis of [Intermediate 9-f]

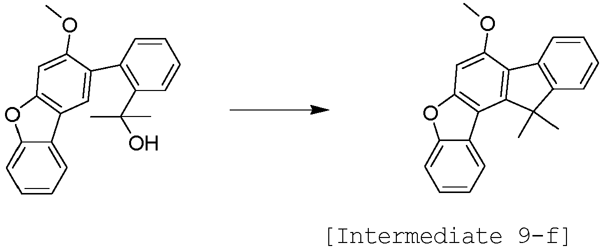

The same procedure as in Synthesis Example 1-3 was carried out, with the exception of using Intermediate 9-e instead of Intermediate 1-b, to afford Intermediate 9-f: yield 67%

### Synthesis Example 9-7: Synthesis of [Intermediate 9-g]

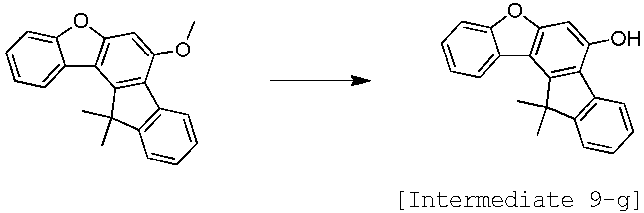

In a round-bottom flask, Intermediate 9-f (30 g, 95.4 mmol) and dichloromethane (200 ml) were mixed and cooled to 0°C. A dilution of brontribromide (120 g, 143 mmol) in dichloromethane (300 mL) was dropwise added slowly into the flask and then stirred at room temperature for 3 hours. After completion of the reaction, the reaction mixture was poured into distilled water (1 L) and the organic layer thus formed was concentrated in a vacuum and separated by column chromatography to afford Intermediate 9-g (19.2 g): yield 67%

### Synthesis Example 9-8: Synthesis of [Intermediate 9-h]

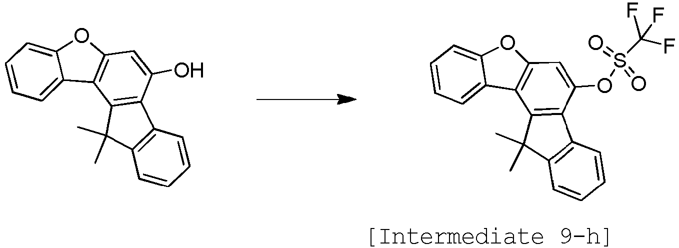

In a round-bottom flask, Intermediate 9-g (10 g, 33.2 mmol) was cooled to 0°C under a nitrogen atmosphere. Pyridine (3.2 ml, 39.9 mmol) and trifluoromethane sulfonic anhydride (7.3 ml, 43.2 mmol) were dropwise added slowly, followed by stirring at room temperature for 2 hours. After completion of the reaction, the organic layer was concentrated in a vacuum. Column chromatographic separation afforded Intermediate 9-h (12.2 g): yield 85%

### Synthesis Example 9-9 (Reference): Synthesis of [Compound 181]

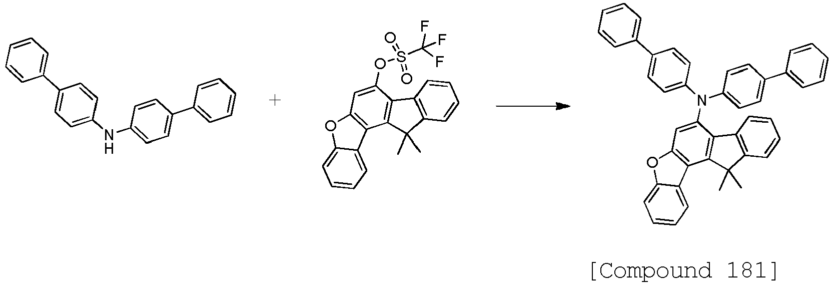

Intermediate 9-h (17.3 g, 40 mmol), bis(4-biphenyl)amine (19.3 g, 60 mmol), palladium acetate (0.5 g, 2 mmol), BINAP (1.9 g, 3 mmol), sodium tert-butoxide (5.8 g, 60 mmol), and toluene (200 ml) were fed into a round-bottom flask under a nitrogen atmosphere, stirred at room temperature for 30 min, and heated at 120°C for 12 hours under reflux. After completion of the reaction, the organic layer was concentrated in a vacuum. Column chromatographic separation afforded Compound 181 (14.7 g): yield 61%
MS (MALDI-TOF): m/z 603.26[M]+

### SYNTHESIS EXAMPLE 10 (Reference): Synthesis of [Compound 183]

### Synthesis Example 10-1: Synthesis of [Intermediate 10-a]

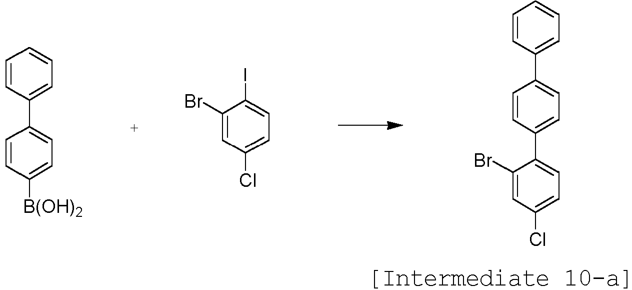

The same procedure as in Synthesis Example 1-1 was carried out, with the exception of using biphenyl-4-boronic acid and 2-bromo-4-chloroiodobenzene instead of dibenzofuran-1-boronic acid and methyl-5-bromo-2-iodobenzoate, respectively, to afford Intermediate 10-a: yield 74%

### Synthesis Example 10-2: Synthesis of [Intermediate 10-b]

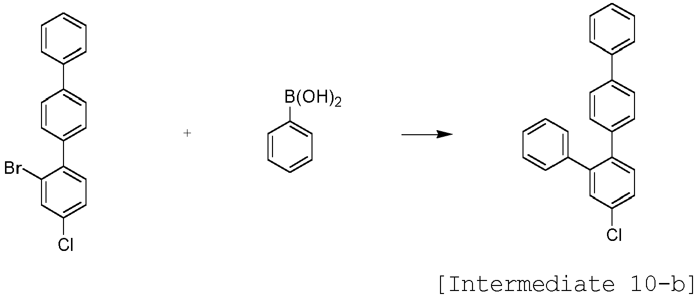

The same procedure as in Synthesis Example 1-1 was carried out, with the exception of using phenyl boronic acid and Intermediate 10-a instead of dibenzofuran-1-boronic acid and methyl-5-bromo-2-iodobenzoate, to afford [Intermediate 10-b]: yield 75%

### Synthesis Example 10-3: Synthesis of [Intermediate 10-c]

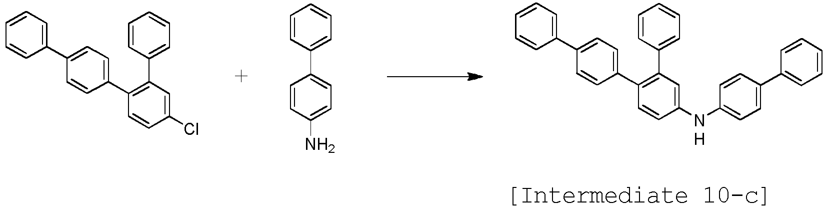

The same procedure as in Synthesis Example 4-2 was carried out, with the exception of using 4-aminobiphenyl and Intermediate 10-b instead of Intermediate 4-a and 4-bromobiphenyl, respectively, to afford Intermediate 10-c: yield 54%

### Synthesis Example 10-4 (Reference): Synthesis of [Compound 183]

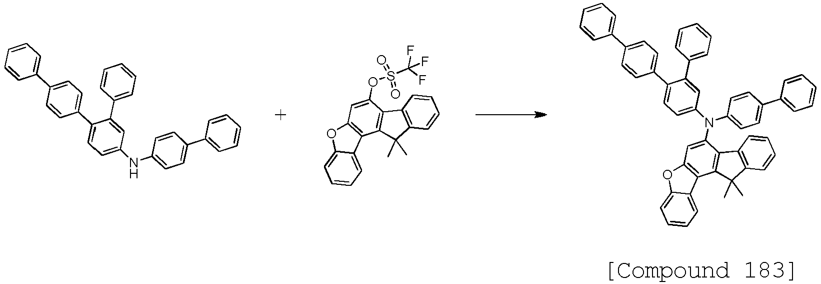

The same procedure as in Synthesis Example 9-9 was carried out, with the exception of using Intermediate 10-c instead of bis(4-biphenyl)amine, to afford Compound 183: yield 67%
MS (MALDI-TOF): m/z 755.32[M]+

### SYNTHESIS EXAMPLE 11 (Reference): Synthesis of [Compound 238]

### Synthesis Example 11-1: Synthesis of [Intermediate 11-a]

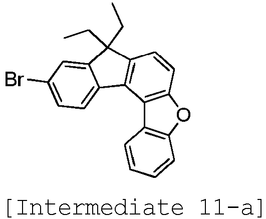

The same procedures as in Synthesis Examples 1-2 to 1-3 were carried out, with the exception of using 3 M-ethyl magnesium bromide instead of 3 M-methyl magnesium bromide in Synthesis Example 1-2, to afforded Intermediate 11-a: yield 67%

### Synthesis Example 11-2 (Reference): Synthesis of [Compound 238]

The same procedure as in Synthesis Example 1-4 was carried out, with the exception of using bis(3-biphenylyl)amine and Intermediate 11-a instead of bis(4-biphenyl)amine and Intermediate 1-c, respectively, to afford [Compound 238]: yield 75%
MS (MALDI-TOF): m/z 631.29 [M]+

### SYNTHESIS EXAMPLE 12 (Reference): Synthesis of [Compound 254]

### Synthesis Example 12-1: Synthesis of [Intermediate 12-a]

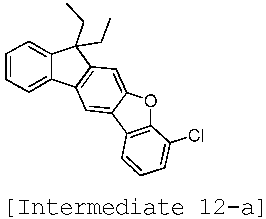

The same procedures as in Synthesis Examples 3-1 to 3-6 were carried out, with the exception of using 2-bromo-9,9-diethylfluorene instead of 2-bromo-9,9-dimethylfluorene in Synthesis Example 3-1, to afford Intermediate 12-a: yield 70%

### Synthesis Example 12-2 (Reference): Synthesis of [Compound 254]

The same procedure as in Synthesis Example 1-4 was carried out, with the exception of using N-phenyl-4-biphenylamine and Intermediate 12-a instead of bis(4-biphenyl)amine and Intermediate 1-c, to afford Compound 254: yield 75%
MS (MALDI-TOF): m/z 555.26 [M]+

### SYNTHESIS EXAMPLE 13 (Reference): Synthesis of [Compound 262]

### Synthesis Example 13-1: Synthesis of [Intermediate 13-a]

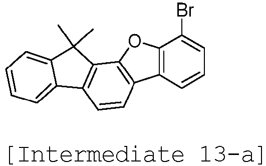

The same procedures as in Synthesis Examples 5-2 to 5-4 was carried out, with the exception of using 2,4-dimethyl-3-pentanone instead of acetone in Synthesis Example 5-2, to afford Intermediate 13-a: yield 50%

### Synthesis Example 13-2 (Reference): Synthesis of [Compound 262]

The same procedure as in Synthesis Example 1-4 was carried out, with the exception of using 4-methyldiphenylamine and 13-a instead of bis(4-biphenyl)amine and Intermediate 1-c, respectively, to afford Compound 262: yield 72%
MS (MALDI-TOF): m/z 610.30 [M]+

### SYNTHESIS EXAMPLE 14 (Reference): Synthesis of [Compound 268]

### Synthesis Example 14-1: Synthesis of [Intermediate 14-a]

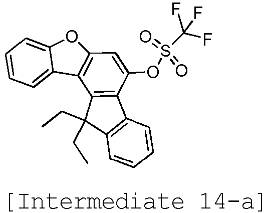

The same procedures as in Synthesis Examples 9-6 to 9-8 were carried out, with the exception of using 3 M-ethyl magnesium bromide instead of 3 M-methyl magnesium bromide in Synthesis Example 1-2 and Intermediate 9-d instead of Intermediate 1-a, to afford Intermediate 14-a: yield 55%

### Synthesis Example 14-2 (Reference): Synthesis of [Compound 268]

The same procedure as in Synthesis Example 1-4 was carried out, with the exception of using N-(4-biphenyl)-(9,9-dimethylfluoren-2-yl)amine and Intermediate 14-a instead of bis(4-biphenyl)amine and Intermediate 1-c, respectively, to afford Compound 268: yield 74%
MS (MALDI-TOF): m/z 671.32 [M]+

### SYNTHESIS EXAMPLE 15 (Reference): Synthesis of [Compound 317]

### Synthesis Example 15-1: Synthesis of [Intermediate 15-a]

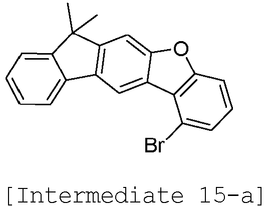

The same procedure as for Intermediate 306 in Synthesis Example 3-6 was carried out, with the exception of using 1-bromo-2-iodo-3-fluorobenzene instead of 1-bromo-3-chloro-2-fluorobenzene in Synthesis Example 3-4, to afford Intermediate 15-a: yield 52%

### Synthesis Example 15-2 (Reference): Synthesis of [Compound 317]

The same procedure as in Synthesis Example 1-4 was carried out, with the exception of using N-phenyl-4-biphenylamine and Intermediate 15-a instead of bis(4-biphenyl)amine and Intermediate 1-c, to afford Compound 317: yield 74%
MS (MALDI-TOF): m/z 671.32 [M]⁺

### SYNTHESIS EXAMPLE 16: Synthesis of [Compound 321]

### Synthesis Example 16-1: Synthesis of [Intermediate 16-a]

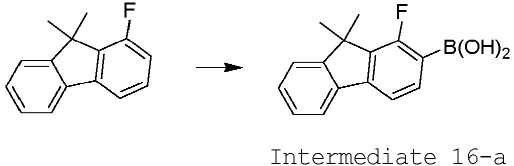

In a round-bottom flask was placed 1-fluoro-9,9'-dimethylfluorene (50 g, 235 mmol) to which tetrahydrofuran (500 ml) was then added under a nitrogen atmosphere. The mixture was cooled to -78°C and slowly added with drops of 1.6 M butyl lithium (161 ml, 259 mmol). The temperature was elevated to room temperature before stirring for 4 hours. Then, the temperature was lowered again to -78°C, and trimethylborate (32 ml, 282 mmol) was slowly added while the low temperature was maintained. Following temperature elevation to room temperature, the reaction mixture was stirred. After completion of the reaction, the organic layer was concentrated in a vacuum and recrystallized in hexane to afford Intermediate 16-a (45 g): yield 75%.

### Synthesis Example 16-2: Synthesis of [Intermediate 16-b]

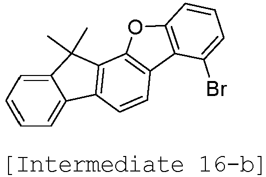

The same procedure as for Intermediate 3-f in Synthesis Example 3-6 was carried out, with the exception of using 1-bromo-2-iodo-3-fluorobenzene instead of 1-bromo-3-chloro-2-fluorobenzene in Synthesis Example 3-4, to afford Intermediate 16-b: yield 50%

### Synthesis Example 16-3: Synthesis of [Compound 321]

The same procedure as in Synthesis Example 1-4 was carried out, with the exception of using N-phenyl-4-biphenylamine and Intermediate 16-b instead of bis(4-bipheneyl)amine and Intermediate 1-c in Synthesis Example 1-4, respectively, to afford Compound 321: yield 71%

### SYNTHESIS EXAMPLE 17 (Reference): Synthesis of [Compound 325]

### Synthesis Example 17-1: Synthesis of [Intermediate 17-a]

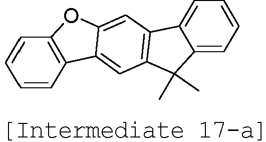

The same procedures as in Synthesis Examples 1-1 and 1-3 were carried out, with the exception of using dibenzofuran-3-boronic acid and methyl-2-bromobenzoate instead of dibenzofuran-1-boronic acid and methyl-2-bromobenzoate in Synthesis Example 1-1, respectively, to afford Intermediate 17-a: yield 70%

### Synthesis Example 17-2: Synthesis of [Intermediate 17-b]

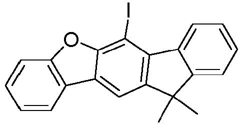

The same procedure as in Synthesis Example 16-1 was carried out, with the exception of using Intermediate 17-a instead of 1-fluoro-9,9'-dimethylfluorene, to afford Intermediate 17-b: yield 74%

### Synthesis Example 17-3 (Reference): Synthesis of [Compound 325]

The same procedure as in Synthesis Example 1-4 was carried out, with the exception of using N-phenyl-4-biphenylamine and Intermediate 17-b instead of bis(4-biphenyl)amine and Intermediate 1-c, respectively, to afford Compound 325: yield 71%

### EXAMPLES 1 TO 17: Fabrication of Organic Light-Emitting Diode

An ITO glass substrate was patterned to have a translucent area of 2 mm x 2 mm and cleansed. The ITO glass was mounted in a vacuum chamber that was then set to have a base pressure of 133×10⁻⁶ Pascal. On the ITO glass substrate, HATCN (50 Å) was deposited, followed by one of the compounds prepared according to the present disclosure, as listed in Table 1, below, for a hole transport layer (650 Å). Then, a light-emitting layer 200 Å thick was formed by doping with 5 % of a blue host (BH) + blue dopant (BD). Subsequently, deposition was made with compound ET:Liq = 1:1 (300Å) for an electron transport layer, Liq (10 Å) for an electron injection layer, and Al (1,000Å). The light-emitting diodes thus obtained were measured at 0.4 mA for luminescence properties. Structures of [HATCN], [BH], [BD], [ET], and [Liq] are as follows:

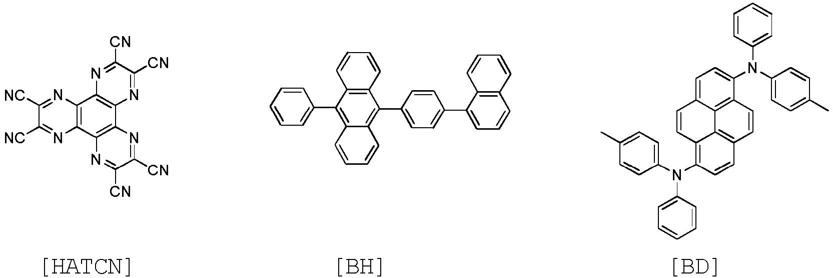

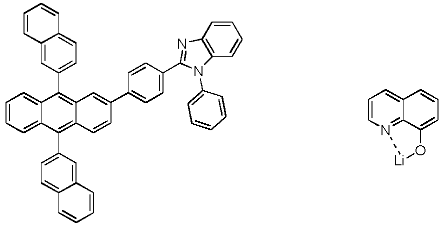

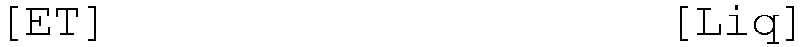

### COMPARATIVE EXAMPLE 1

An organic light-emitting diode was fabricated in the same manner as in Examples 1 to 17, with the exception of using, instead of the compounds prepared in the present disclosure, NPD which is conventionally widely used as a hole transport layer material.

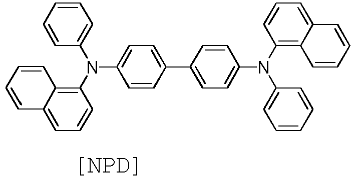

The organic EL devices fabricated in the Examples 1 to 17 and Comparative Example 1 were measured for voltage, luminance, color coordinates, and lifespan, and the results are summarized in Table 1, below.

Here, T95 refers to the time for luminance to decrease to 95 % of the initial luminance (2000 cd/m2).

**TABLE 1**

| | HTL | V | Cd/A | CIEx | CIEy | T95 (Hrs) |
|---|---|---|---|---|---|---|
| C. Ex. 1 | NPD | 4.2 | 6.3 | 0.133 | 0.129 | 13 |
| Ex. 1* | Chemical Formula 1 | 3.6 | 8.0 | 0.132 | 0.130 | 35 |
| Ex. 2* | Chemical Formula 3 | 3.7 | 7.8 | 0.133 | 0.128 | 33 |
| Ex. 3* | Chemical Formula 67 | 3.4 | 8.0 | 0.132 | 0.126 | 34 |
| Ex. 4* | Chemical Formula 68 | 3.6 | 7.9 | 0.133 | 0.125 | 37 |
| Ex. 5* | Chemical Formula 111 | 3.8 | 8.0 | 0.133 | 0.125 | 43 |
| Ex. 6* | Chemical Formula 120 | 3.7 | 7.9 | 0.132 | 0.126 | 40 |
| Ex. 7* | Chemical Formula 136 | 3.7 | 7.8 | 0.132 | 0.130 | 34 |
| Ex. 8* | Chemical Formula 141 | 3.5 | 7.7 | 0.133 | 0.128 | 34 |
| Ex. 9* | Chemical Formula 181 | 3.6 | 8.1 | 0.133 | 0.125 | 40 |
| Ex. 10* | Chemical Formula 183 | 4.0 | 7.8 | 0.132 | 0.129 | 35 |
| Ex. 11* | Chemical Formula 238 | 3.8 | 7.7 | 0.132 | 0.130 | 35 |
| Ex. 12* | Chemical Formula 254 | 3.6 | 7.7 | 0.133 | 0.130 | 38 |
| Ex. 13* | Chemical Formula 262 | 3.7 | 7.8 | 0.133 | 0.128 | 36 |
| Ex. 14* | Chemical Formula 268 | 3.6 | 7.9 | 0.132 | 0.129 | 40 |
| Ex. 15* | Chemical Formula 317 | 3.6 | 7.8 | 0.133 | 0.126 | 60 |
| Ex. 16 | Chemical Formula 321 | 3.7 | 7.9 | 0.132 | 0.129 | 55 |
| Ex. 17 | Chemical Formula 324 | 3.6 | 8.0 | 0.133 | 0.125 | 56 |

| | | | | | | |
|---|---|---|---|---|---|---|
| *Reference Examples not included within the scope of the present invention | | | | | | |

Compared to the conventional hole transport layer material NPD, the organic compounds prepared according to the present disclosure guarantee higher efficiency, lower driving voltages, and longer lifespans for the OLED, as understood from the data of Table 1, and thus can be applied to the fabrication of organic light-emitting diodes having improved properties.

Particularly, more improved lifespan properties were detected in Examples 15 to 17 than the other Examples of Examples 1 to 17 (Examples 1 to 15, 17 being Reference Examples).

### COMPARATIVE EXAMPLE 2: Fabrication of Organic Light-Emitting Diode

An ITO glass substrate was patterned to have a translucent area of 2 mm x 2 mm and cleansed. The ITO glass substrate was mounted in a vacuum chamber that was then set to have a base pressure of 133×10⁻⁶ Pascal On the ITO glass substrate, HATCN (50 Å) was deposited, followed by NPD (900 Å). Then, a light-emitting layer 300 Å thick was formed by doping the host material CBP and a green phosphorescent dopant (GD, 7 %). Subsequently, deposition was made with compound ET:Liq = 1:1 (300Å), Liq (10 Å), and Al (1,000Å) in that order. The light-emitting diode thus obtained were measured at 0.4 mA for luminescence properties.

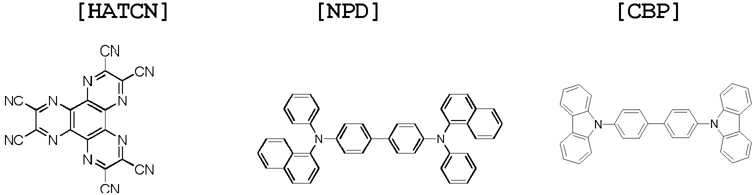

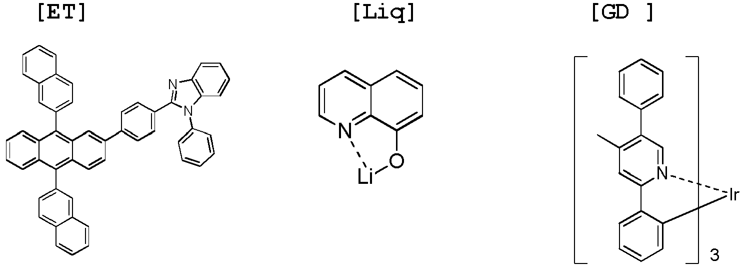

### COMPARATIVE EXAMPLE 3

An electroluminescent device was fabricated according to a conventional method using Comparative Compound 1 as a hole auxiliary layer material. First, an ITO glass substrate was patterned to have a translucent area of 2 mm x 2 mm and cleansed. The ITO glass substrate was mounted in a vacuum chamber that was then set to have a base pressure of 133x10⁻⁶ Pascal. On the ITO glass substrate, HATCN (50 Å) was deposited, followed by NPD (550 Å). Then, a hole auxiliary layer was formed on the hole transport layer by depositing the Comparative Compound 1 (350 Å), and a light-emitting layer 300 Å thick was formed by doping the host material CBP and a green phosphorescent dopant (GD, 7 %). Subsequently, deposition was made with compound ET:Liq = 1:1 (300Å), Liq (10 Å), and Al (1,000Å) in that order. The light-emitting diode thus obtained were measured at 0.4 mA for luminescence properties.

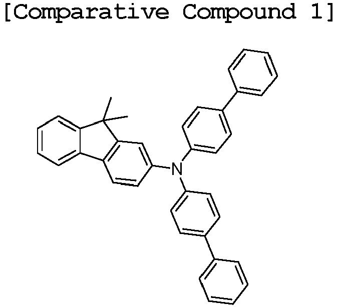

### REFERENCE EXAMPLES 18 TO 21, and Examples 22 and 23

Organic light-emitting diodes were fabricated in the same manner as in Comparative Examples 2, with the exception of using the compounds of the present disclosure, instead of Comparative Material 1, as a hole auxiliary layer material.

**TABLE 2**

| | **Hole auxiliary layer** | **V** | **Cd/A** | **CIEx** | **CIEy** | **T₉₅ (Hrs)** |
|---|---|---|---|---|---|---|
| C. Ex. 2 | - | 6.9 | 36 | 0.335 | 0.625 | 20 |
| C. Ex. 3 | C. Compound 1 | 6.9 | 42 | 0.329 | 0.631 | 25 |
| Ex. 18* | Chemical Formula 67 | 6.9 | 51 | 0.327 | 0.632 | 41 |
| Ex. 19* | Chemical Formula 111 | 6.8 | 49 | 0.330 | 0.631 | 45 |
| Ex. 20* | Chemical Formula 181 | 7.0 | 50 | 0.337 | 0.625 | 43 |
| Ex. 21* | Chemical Formula 317 | 6.9 | 52 | 0.329 | 0.631 | 71 |
| Ex. 22 | Chemical Formula 321 | 6.8 | 53 | 0.327 | 0.632 | 65 |
| Ex. 23 | Chemical Formula 324 | 6.9 | 51 | 0.329 | 0.631 | 68 |

| | | | | | | |
|---|---|---|---|---|---|---|
| *Reference Examples not included within the scope of the present invention | | | | | | |

When used in a hole auxiliary layer, as can be seen in Table 2, the compounds obtained according to the present invention guarantee higher efficiency and longer lifespan for the device than do the compounds of Comparative Examples 2 and 3, and thus can be applied to the fabrication of organic light-emitting devices having improved characteristics.

Particularly, compounds of Examples 21 to 23 were observed to have improved longevity properties compared to the other Examples.

According to the present disclosure, the organic light-emitting diode containing the compound according to claim 1 in a hole transport layer thereof exhibits the outstanding properties of high efficiency, low voltage, and long lifespan, compared to existing organic light-emitting diodes.

## Claims

1. An organic compound represented by Chemical Formula A or Chemical Formula B: 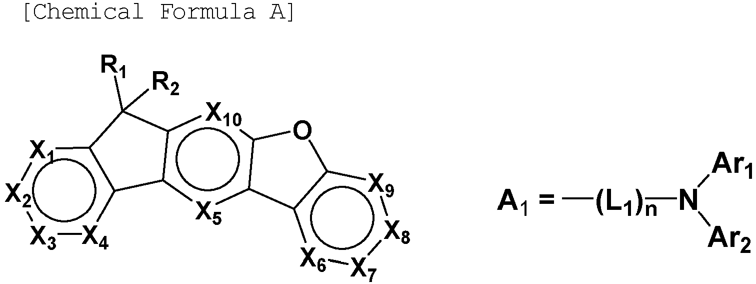 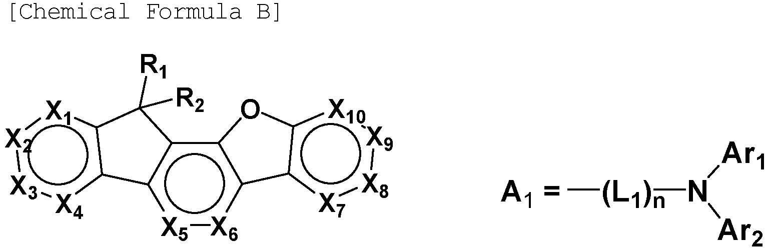 wherein,
X1 is CR11 or N,
X2 is CR12 or N,
X3 is CR13 or N,
X4 is CR14 or N,
X5 is CR15 or N,
X6 is CR16 or N,
X7 is CR17 or N,
X8 is CR18 or N,
X9 is CR19 or N, and
X10 is CR20 or N, wherein at least two of X1 to X4 are selected from among CR11 to CR14 and at least three of X5 to X10 are selected from among CR15 to CR20, with a proviso that one of X1 to X10 is a carbon atom connected to A1 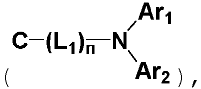
the substituents R1, R2, and R11 to R20, which may be the same or different, are each independently selected from among a hydrogen, a deuterium, a substituted or unsubstituted alkyl of 1 to 30 carbon atoms, a substituted or unsubstituted cycloalkyl of 3 to 30 carbon atoms, a substituted or unsubstituted aryl of 6 to 50 carbon atoms, a substituted or unsubstituted heteroaryl of 2 to 50 carbon atoms bearing O, N, or S as a heteroatom, an alkylsilyl of 1 to 24 carbon atoms, an arylsilyl of 6 to 24 carbon atoms, a nitro and a halogen,
L1 denotes a linker and is any one selected from among a single bond, a substituted or unsubstituted arylene of 6 to 60 carbon atoms, and a substituted or unsubstituted heteroarylene of 2 to 60 carbon atoms,
n is an integer of 0 to 3, with a proviso that when n is 2 or greater, the corresponding L1 linkers may be the same or different, and
Ar1 and Ar2, which may be the same or different, are each independently any one selected from among a substituted or unsubstituted aryl of 6 to 40 carbon atoms and a substituted or unsubstituted heteroaryl of 2 to 30 carbon atoms, with a proviso that Ar1 may be connected to L1 to form a fused ring and Ar2 may be connected to L1 to form a fused ring in A1,
wherein the organic compound represented by Chemical Formula A or Chemical Formula B has A1 bonded to position 1 of the dibenzofuran moiety thereof in such a manner that A1 is bonded to the carbon atom of X6 in Chemical Formula A and X7 in Chemical Formula B,
wherein the term 'substituted' in the expression 'substituted or unsubstituted' means having at least one substituent selected from the group consisting of a deuterium, a cyano, a halogen, a nitro, an alkyl of 1 to 24 carbon atoms, a halogenated alkyl of 1 to 24 carbon atoms, an aryl of 6 to 24 carbon atoms, an arylalkyl of 6 to 24 carbon atoms, a heteroaryl of 2 to 24 carbon atoms or a heteroarylalkyl of 2 to 24 carbon atoms, an alkylsilyl of 1 to 24 carbon atoms and an arylsilyl of 1 to 24 carbon atoms,
wherein compounds 301, 302, 304, 305, 307, 317, 319 and 320 are excluded:

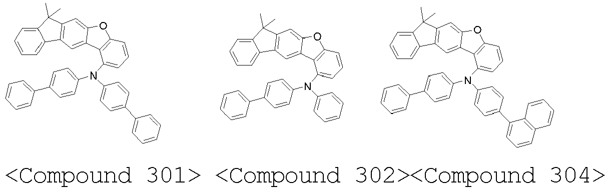

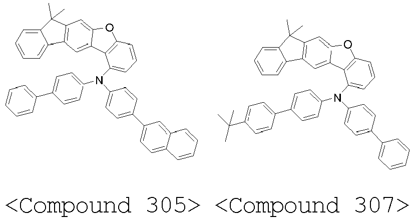

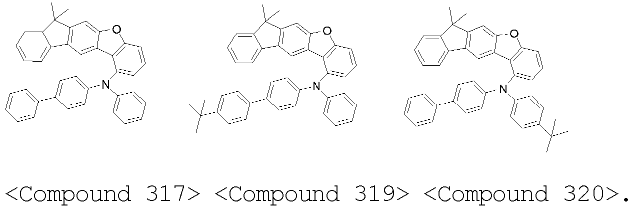

2. The organic compound as set forth in claim 1, wherein Ar1 and Ar2 in Chemical Formula A and Chemical Formula B may be the same or different and are each independently a substituted or unsubstituted aryl of 6 to 30 carbon atoms.

3. The organic compound as set forth in claim 1, wherein R11 to R20 on the carbon atoms which are not directly connected to A1 in Chemical Formula A and Chemical Formula B are each a hydrogen atom or a deuterium atom.

4. The organic compound as set forth in claim 1, wherein the linker L1 in Chemical Formula A and Chemical Formula B is a single bond or any one selected from among the following Structural Formulas 1 to 9: 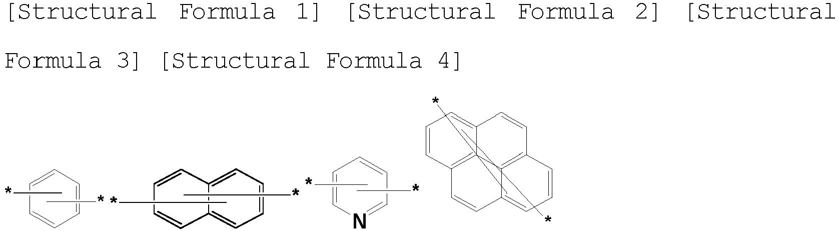 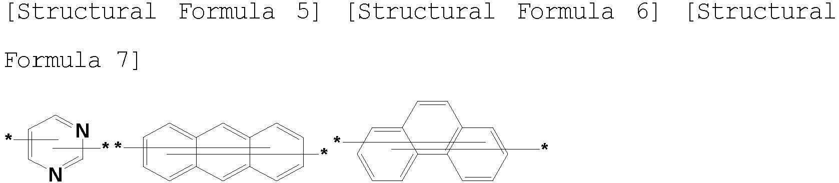 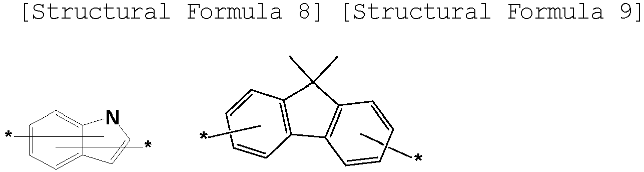 wherein carbon atoms in the aromatic ring moieties are bonded to a hydrogen atom or a deuterium atom.

5. The organic compound as set forth in claim 1, wherein n in Chemical Formula A and Chemical Formula B is an integer of 1 or 2, with a proviso that when n is 2, the corresponding L1 linkers may be the same or different.

6. The organic compound as set forth in claim 1, wherein only one or none of X1 to X10 are a nitrogen atom.

7. The organic compound as set forth in claim 1, wherein the organic compound represented by any one of Chemical Formula A and Chemical Formula B is selected from among the compounds represented by the following Compounds 81 to 85, Compounds 126 to 130, Compound 252, Compound 303, Compound 306, Compounds 308 to 316, Compound 318, Compounds 321 to 324:

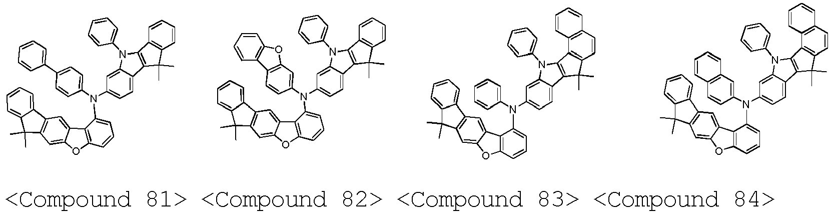

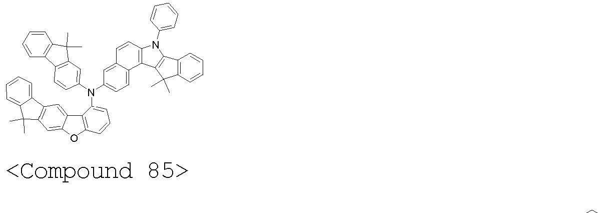

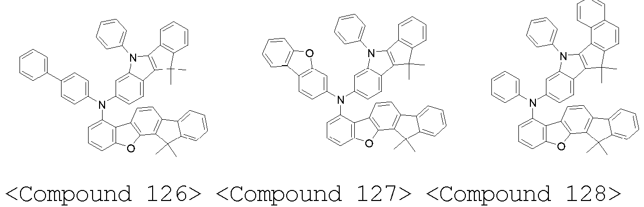

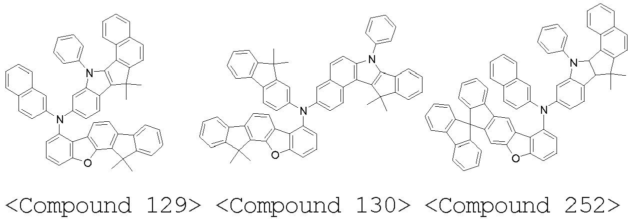

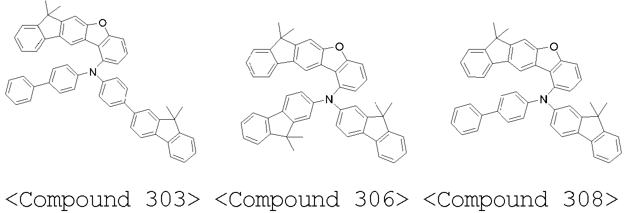

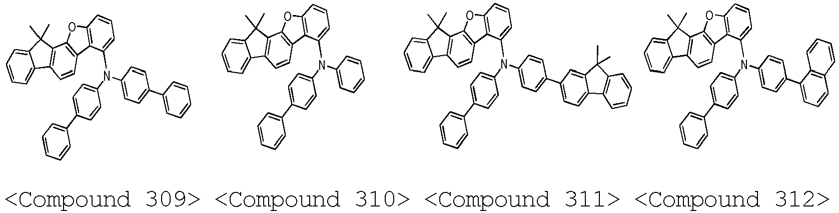

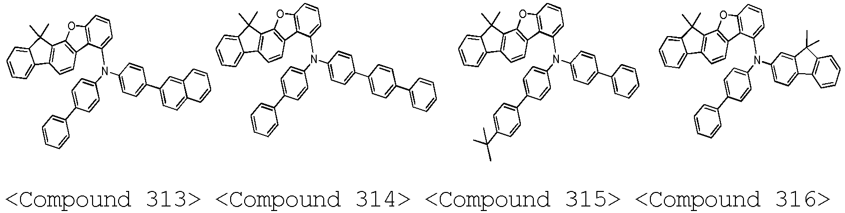

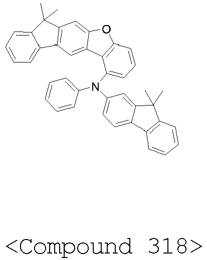

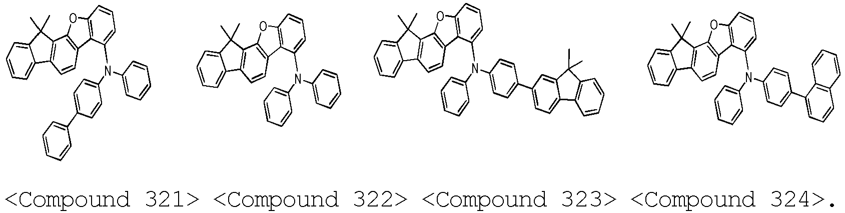

8. An organic light-emitting diode, including:
a first electrode;
a second electrode facing the first electrode; and
an organic layer interposed the first electrode and the second electrode,
wherein the organic layer contains the organic compound of any one of claims 1 to 7.

9. The organic light-emitting diode as set forth in claim 8, wherein the organic layer includes at least one of a hole injection layer, a hole transport layer, a functional layer capable of both hole injection and hole transport, a light-emitting layer, an electron transport layer, and an electron injection layer.

10. The organic light-emitting diode as set forth in claim 8, wherein the organic compound is used for a hole transport layer.

11. The organic light-emitting diode as set forth in claim 9, wherein at least one selected from among the layers is deposited using a deposition process or a solution process.

12. The organic light-emitting diode as set forth in claim 8, wherein the organic light-emitting diode is used for a device selected from among a flat display device, a flexible display device, a monochrome or grayscale flat illumination device, and a monochrome or grayscale flexible illumination device.

## Patentansprüche

1. Eine organische Verbindung, die durch die chemische Formel A oder die chemische Formel B dargestellt wird: 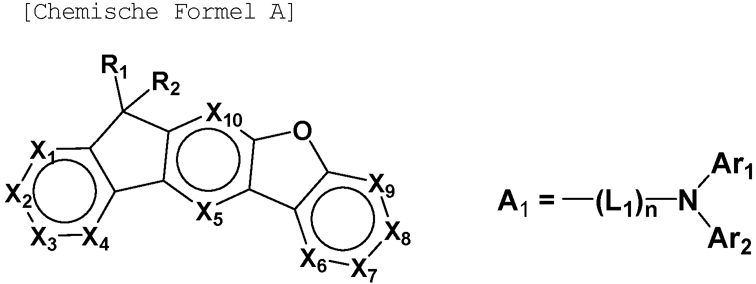 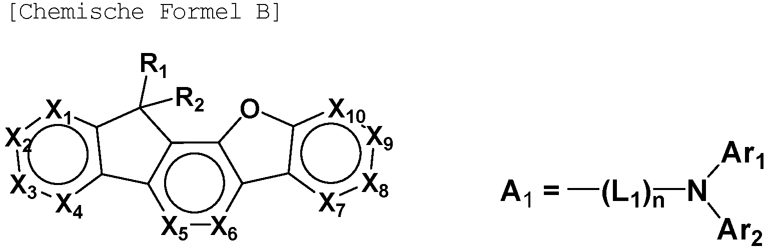 wobei,
X1 CR11 oder N ist,
X2 CR12 oder N ist,
X3 CR13 oder N ist,
X4 CR14 oder N ist,
X5 CR15 oder N ist,
X6 CR16 oder N ist,
X7 CR17 oder N ist,
X8 CR18 oder N ist,
X9 CR19 oder N ist, und
X10 CR20 oder N ist, wobei mindestens zwei von X1 bis X4 ausgewählt sind aus CR11 bis CR14 und mindestens drei von X5 bis X10 ausgewählt sind aus CR15 bis CR20, mit der Maßgabe, dass eines von X1 bis X10 ein mit A1 verbundenes Kohlenstoffatom ist ( 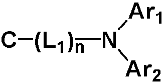 ),
die Substituenten R1, R2 und R11 bis R20, die gleich oder verschieden sein können, jeweils unabhängig voneinander ausgewählt sind aus einem Wasserstoff, einem Deuterium, einem substituierten oder unsubstituierten Alkyl mit 1 bis 30 Kohlenstoffatomen, einem substituierten oder unsubstituierten Cycloalkyl mit 3 bis 30 Kohlenstoffatomen einem substituierten oder unsubstituierten Aryl mit 6 bis 50 Kohlenstoffatomen, einem substituierten oder unsubstituierten Heteroaryl mit 2 bis 50 Kohlenstoffatomen, das O, N oder S als Heteroatom trägt, einem Alkylsilyl mit 1 bis 24 Kohlenstoffatomen, einem Arylsilyl mit 6 bis 24 Kohlenstoffatomen, einem Nitro und einem Halogen,
L1 einen Linker bezeichnet und ausgewählt ist aus einer Einfachbindung, einem substituierten oder unsubstituierten Arylen mit 6 bis 60 Kohlenstoffatomen und einem substituierten oder unsubstituierten Heteroarylen mit 2 bis 60 Kohlenstoffatomen,
n eine ganze Zahl von 0 bis 3 ist, mit der Maßgabe, dass, wenn n 2 oder größer ist, die entsprechenden L1-Linker gleich oder unterschiedlich sein können, und
Ar1 und Ar2, die gleich oder verschieden sein können, jeweils unabhängig voneinander ausgewählt sind aus einem substituierten oder unsubstituierten Aryl mit 6 bis 40 Kohlenstoffatomen und einem substituierten oder unsubstituierten Heteroaryl mit 2 bis 30 Kohlenstoffatomen, mit der Maßgabe, dass Ar1 mit L1 verbunden sein kann, um einen kondensierten Ring zu bilden, und Ar2 mit L1 verbunden sein kann, um einen kondensierten Ring in A1 zu bilden,
wobei die organische Verbindung, die durch die chemische Formel A oder die chemische Formel B dargestellt wird, A1 an die Position 1 des Dibenzofurananteils davon in einer solchen Weise gebunden hat, dass A1 an das Kohlenstoffatom von X6 in der chemischen Formel A und X7 in der chemischen Formel B gebunden ist,
wobei der Begriff "substituiert" im Ausdruck "substituiert oder unsubstituiert" mindestens einen Substituenten, der aus der Gruppe ausgewählt ist, die aus einem Deuterium, einer Cyanogruppe, einem Halogen, einer Nitrogruppe, einem Alkyl mit 1 bis 24 Kohlenstoffatomen, einem halogenierten Alkyl mit 1 bis 24 Kohlenstoffatomen, einem Aryl mit 6 bis 24 Kohlenstoffatomen, einem Arylalkyl mit 6 bis 24 Kohlenstoffatomen, einem Heteroaryl mit 2 bis 24 Kohlenstoffatomen oder einem Heteroarylalkyl mit 2 bis 24 Kohlenstoffatomen, einem Alkylsilyl mit 1 bis 24 Kohlenstoffatomen und einem Arylsilyl mit 1 bis 24 Kohlenstoffatomen besteht, aufweisend bedeutet,
wobei die Verbindungen 301, 302, 304, 305, 307, 317, 319 und 320 ausgeschlossen sind:

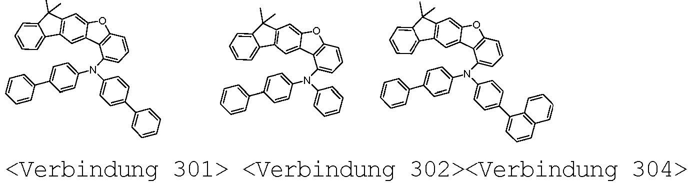

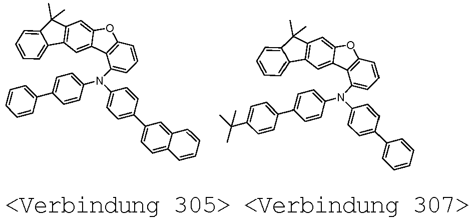

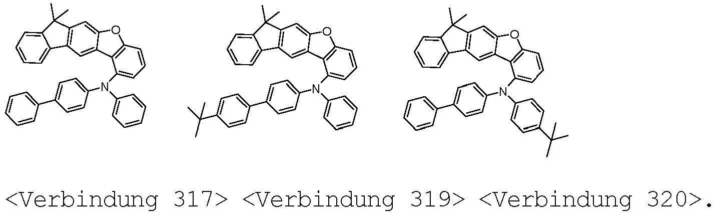

2. Die organische Verbindung nach Anspruch 1, wobei Ar1 und Ar2 in der chemischen Formel A und der chemischen Formel B gleich oder verschieden sein können und jeweils unabhängig voneinander ein substituiertes oder unsubstituiertes Aryl mit 6 bis 30 Kohlenstoffatomen sind.

3. Die organische Verbindung nach Anspruch 1, wobei R11 bis R20 an den Kohlenstoffatomen, die nicht direkt mit A1 in der chemischen Formel A und der chemischen Formel B verbunden sind, jeweils ein Wasserstoffatom oder ein Deuteriumatom sind.

4. Die organische Verbindung nach Anspruch 1, wobei der Linker L1 in der chemischen Formel A und der chemischen Formel B eine Einfachbindung oder eine ist, die aus den folgenden Strukturformeln 1 bis 9 ausgewählt ist: 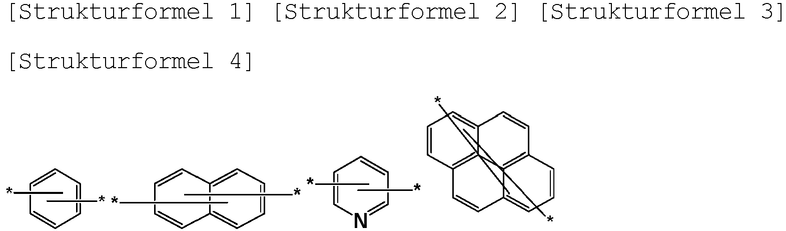 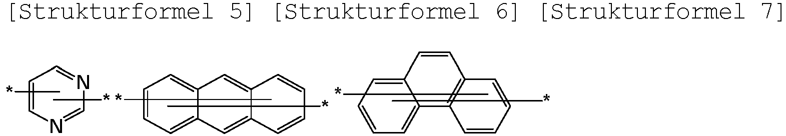 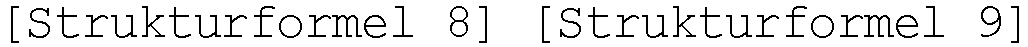 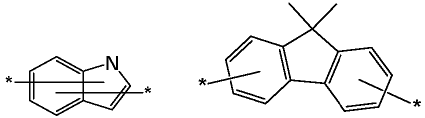 wobei die Kohlenstoffatome in den aromatischen Ringeinheiten an ein Wasserstoffatom oder ein Deuteriumatom gebunden sind.

5. Die organische Verbindung nach Anspruch 1, wobei n in der chemischen Formel A und der chemischen Formel B eine ganze Zahl von 1 oder 2 ist, mit der Maßgabe, dass, wenn n 2 ist, die entsprechenden L1-Linker gleich oder verschieden sein können.

6. Organische Verbindung nach Anspruch 1, wobei nur eines oder keines von X1 bis X10 ein Stickstoffatom ist.

7. Organische Verbindung nach Anspruch 1, wobei die organische Verbindung, die durch eine der chemischen Formeln A und B dargestellt wird, aus den Verbindungen ausgewählt ist, die durch die folgenden Verbindungen 81 bis 85, Verbindungen 126 bis 130, Verbindung 252, Verbindung 303, Verbindung 306, Verbindungen 308 bis 316, Verbindung 318, Verbindungen 321 bis 324 dargestellt sind:

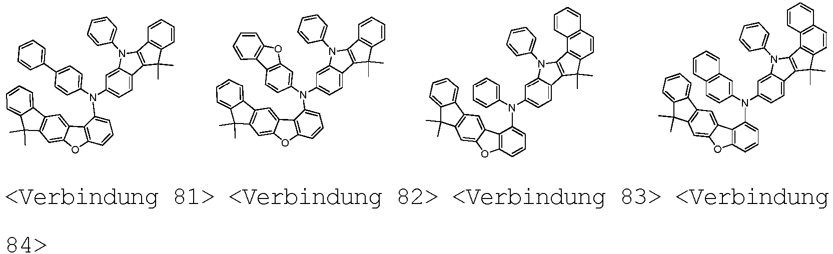

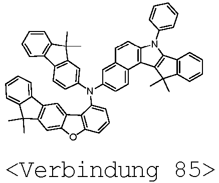

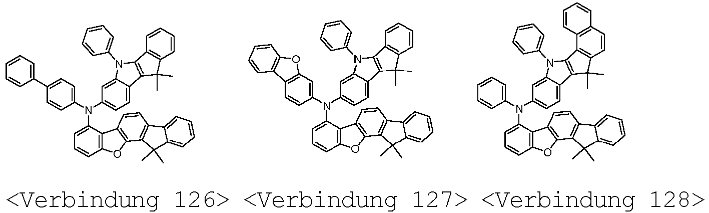

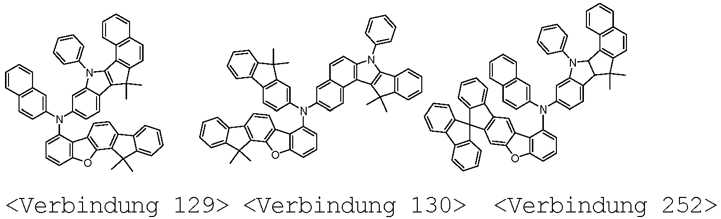

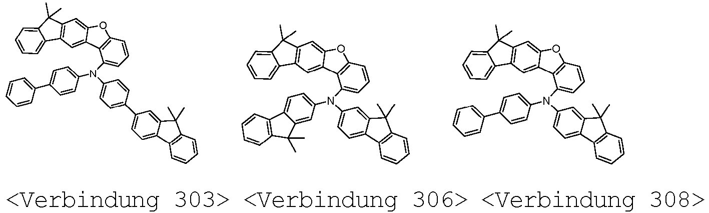

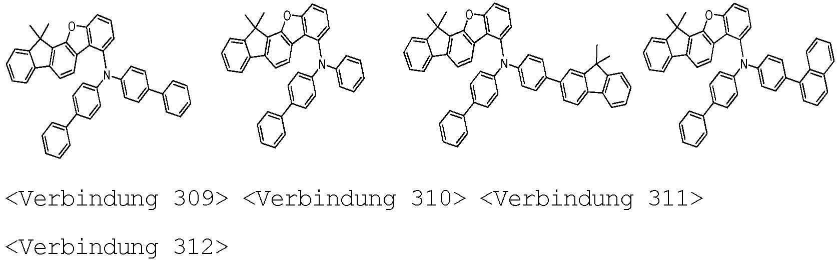

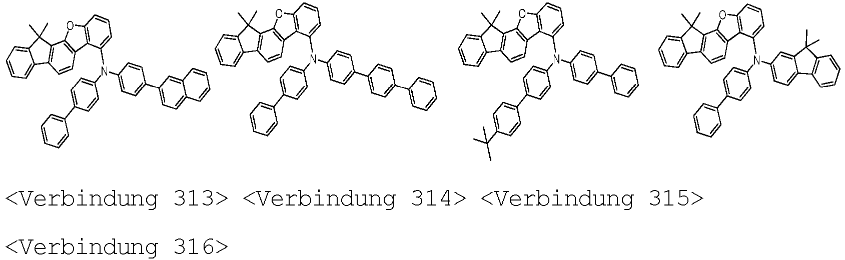

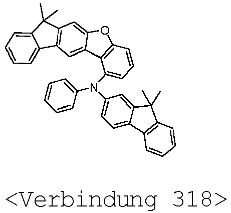

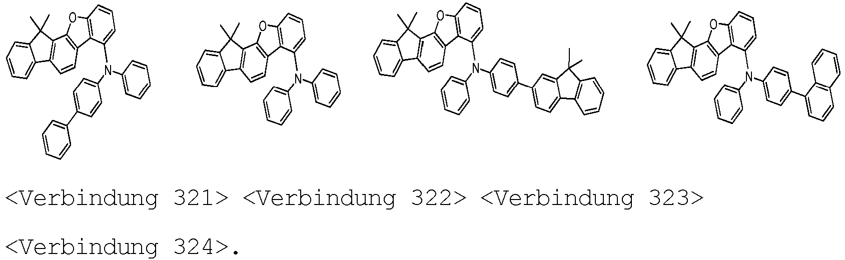

8. Eine organische lichtemittierende Diode, beinhaltend:
eine erste Elektrode;
eine zweite Elektrode, die der ersten Elektrode gegenüberliegt; und
eine organische Schicht, die zwischen der ersten und der zweiten Elektrode liegt,
wobei die organische Schicht die organische Verbindung nach einem der Ansprüche 1 bis 7 enthält.

9. Die organische Leuchtdiode nach Anspruch 8, wobei die organische Schicht mindestens eine von einer Lochinjektionsschicht, einer Lochtransportschicht, einer Funktionsschicht, die sowohl zur Lochinjektion als auch zum Lochtransport fähig ist, einer lichtemittierende Schicht, einer Elektronentransportschicht und einer Elektroneninjektionsschicht beinhaltet.

10. Die organische Leuchtdiode nach Anspruch 8, wobei die organische Verbindung für eine Lochtransportschicht verwendet wird.

11. Die organische Leuchtdiode nach Anspruch 9, wobei zumindest eine ausgewählt aus den Schichten durch ein Abscheideverfahren oder ein Lösungsverfahren abgeschieden wird.

12. Die organische Leuchtdiode nach Anspruch 8, wobei die organische Leuchtdiode für eine Vorrichtung verwendet wird, die aus einer flachen Anzeigevorrichtung, einer flexiblen Anzeigevorrichtung, einer flachen Monochrom- oder Graustufenbeleuchtungsvorrichtung und einer flexiblen Monochrom- oder Graustufenbeleuchtungsvorrichtung ausgewählt ist.

## Revendications

1. Composé organique représenté par la Formule Chimique A ou la Formule Chimique B: 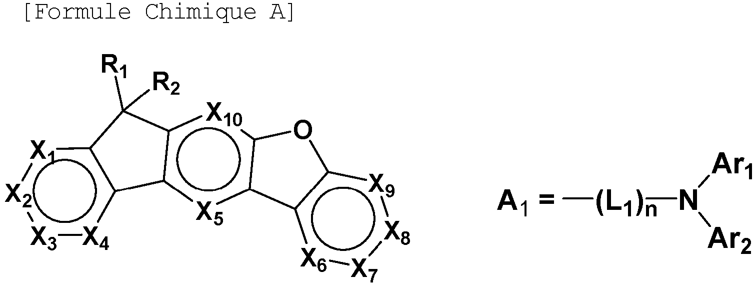 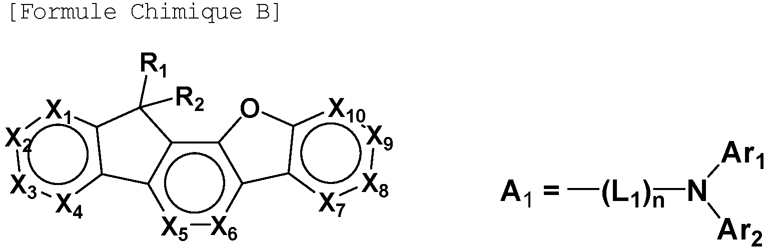 dans lequel,
X1 est CR11 ou N,
X2 est CR12 ou N,
X3 est CR13 ou N,
X4 est CR14 ou N,
X5 est CR15 ou N,
X6 est CR16 ou N,
X7 est CR17 ou N,
X8 est CR18 ou N,
X9 est CR19 ou N, et
X10 est CR20 ou N, dans lequel au moins deux des X1 à X4 sont choisis parmi les CR11 à CR14 et au moins trois des X5 à X10 sont choisis parmi les CR15 à CR20, à condition que l'un des X1 à X10 soit un atome de carbone relié à A1 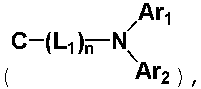
les substituants R1, R2 et R11 à R20, qui peuvent être identiques ou différents, sont chacun indépendamment choisi parmi un hydrogène, un deutérium, un alkyle substitué ou non substitué de 1 à 30 atomes de carbone, un cycloalkyle substitué ou non substitué de 3 à 30 atomes de carbone, un aryle substitué ou non substitué de 6 à 50 atomes de carbone, un hétéroaryle substitué ou non substitué de 2 à 50 atomes de carbone portant O, N ou S comme hétéroatome, un alkylsilyle de 1 à 24 atomes de carbone, un arylsilyle de 6 à 24 atomes de carbone, un nitro et un halogène,
L1 désigne un élément de liaison et est choisi parmi une liaison simple, un arylène substitué ou non substitué de 6 à 60 atomes de carbone et un hétéroarylène substitué ou non substitué de 2 à 60 atomes de carbone,
n est un nombre entier compris entre 0 et 3, à condition que lorsque n est égal ou supérieur à 2, les éléments de liaison L1 correspondants peuvent être identiques ou différents, et
Ar1 et Ar2, qui peuvent être identiques ou différents, sont chacun indépendamment choisi parmi un aryle substitué ou non substitué de 6 à 40 atomes de carbone et un hétéroaryle substitué ou non substitué de 2 à 30 atomes de carbone, à condition que Ar1 peuvent être connecté à L1 pour former un anneau fusionné et que Ar2 peuvent être connecté à L1 pour former un anneau fusionné dans A1,
dans lequel le composé organique représenté par la Formule Chimique A ou la Formule Chimique B a A1 lié à la position 1 de sa fraction dibenzofurane de telle manière que A1 est lié à l'atome de carbone X6 dans la Formule Chimique A et X7 dans la Formule Chimique B,
dans lequel le terme "substitué" dans l'expression "substitué ou non substitué" signifie avoir au moins un substituant choisi dans le groupe consistant en un deutérium, un cyano, un halogène, un nitro, un alkyle de 1 à 24 atomes de carbone, un alkyle halogéné de 1 à 24 atomes de carbone, un aryle de 6 à 24 atomes de carbone, un arylalkyle de 6 à 24 atomes de carbone, un hétéroaryle de 2 à 24 atomes de carbone ou un hétéroarylalkyle de 2 à 24 atomes de carbone, un alkylsilyle de 1 à 24 atomes de carbone et un arylsilyle de 1 à 24 atomes de carbone,
dans lequel les composés 301, 302, 304, 305, 307, 317, 319 et 320 sont exclus:

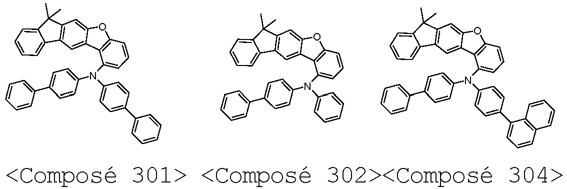

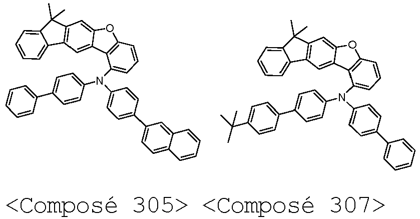

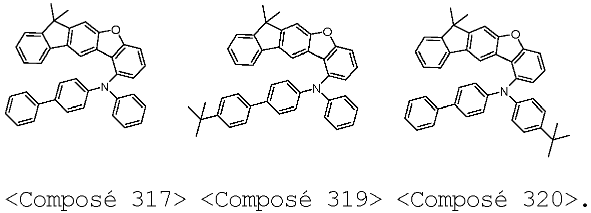

2. Composé organique selon la revendication 1, dans lequel Ar1 et Ar2 dans la Formule Chimique A et la Formule Chimique B peuvent être identiques ou différents et sont chacun indépendamment un aryle substitué ou non substitué de 6 à 30 atomes de carbone.

3. Composé organique selon la revendication 1, dans lequel R11 à R20 sur les atomes de carbone qui ne sont pas directement reliés à A1 dans la Formule Chimique A et la Formule Chimique B sont chacun un atome d'hydrogène ou un atome de deutérium.

4. Composé organique selon la revendication 1, dans lequel l'élément de liaison L1 dans la Formule Chimique A et la Formule Chimique B est une liaison simple ou une liaison choisie parmi les Formules Structurelles 1 à 9 suivantes: 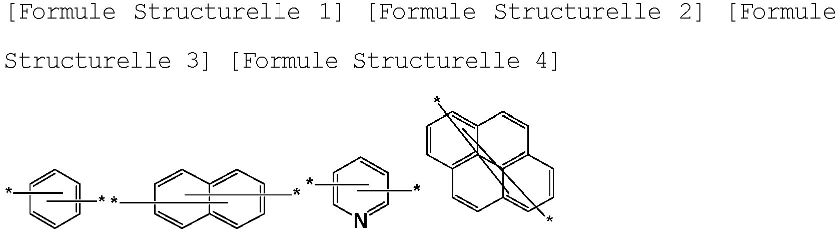 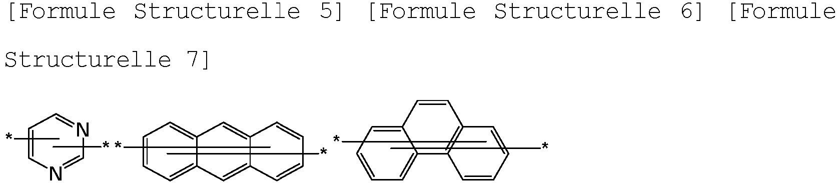 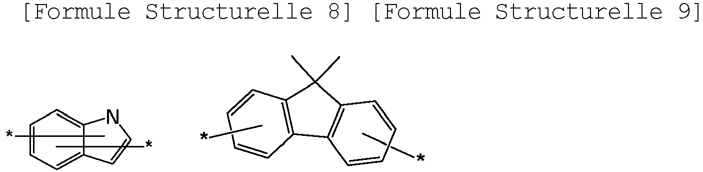 dans lequel les atomes de carbone des fractions du cycle aromatique sont liés à un atome d'hydrogène ou à un atome de deutérium.

5. Composé organique selon la revendication 1, dans lequel n dans la Formule Chimique A et la Formule Chimique B est un nombre entier de 1 ou 2, à condition que lorsque n est 2, les éléments de liaison L1 correspondantes peuvent être identiques ou différentes.

6. Composé organique selon la revendication 1, dans lequel un seul ou aucun des X1 à X10 est un atome d'azote.

7. Composé organique selon la revendication 1, dans lequel le composé organique représenté par l'une quelconque des Formules Chimiques A et B est choisi parmi les composés représentés par les composés 81 à 85, les composés 126 à 130, le composé 252, le composé 303, le composé 306, les composés 308 à 316, le composé 318, les composés 321 à 324 suivantes:

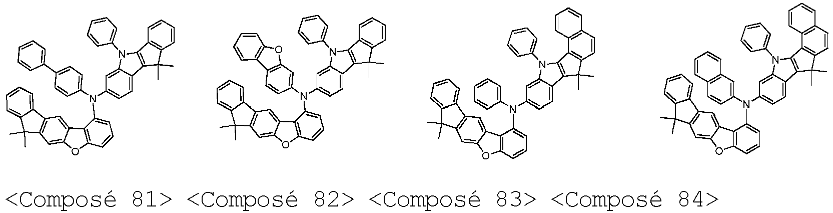

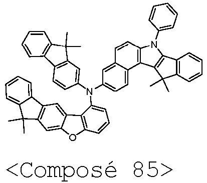

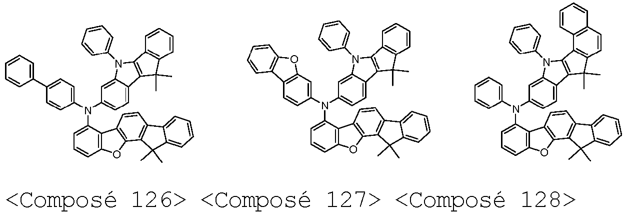

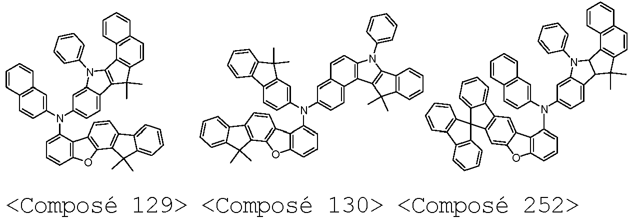

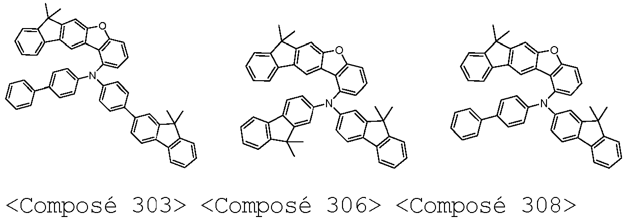

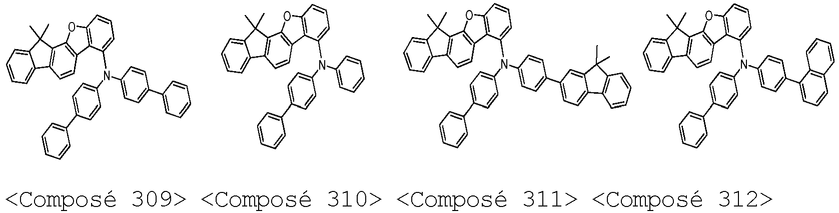

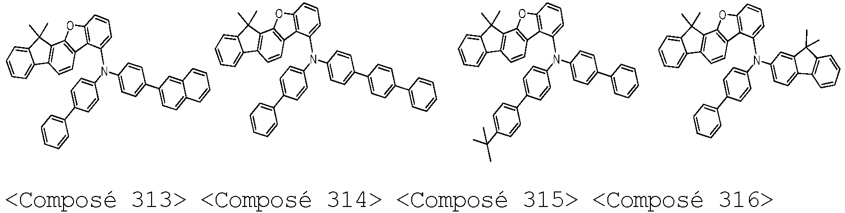

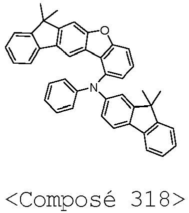

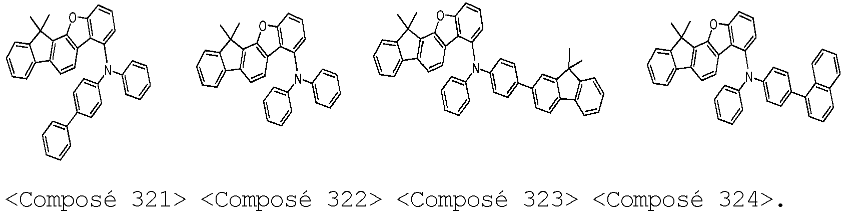

8. Une diode électroluminescente organique, comprenant:
une première électrode ;
une deuxième électrode faisant face à la première électrode ; et
une couche organique interposée entre la première électrode et la seconde électrode,
dans lequel la couche organique contient le composé organique de l'une des revendications 1 à 7.

9. Diode organique électroluminescente selon la revendication 8, dans laquelle la couche organique comprend au moins une couche d'injection de trous, une couche de transport de trous, une couche fonctionnelle capable à la fois d'injecter et de transporter des trous, une couche d'émission de lumière, une couche de transport d'électrons et une couche d'injection d'électrons.

10. Diode organique électroluminescente selon la revendication 8, dans laquelle le composé organique est utilisé pour une couche de transport de trous.

11. Diode organique électroluminescente selon la revendication 9, dans laquelle au moins une des couches sélectionnées est déposée à l'aide d'un procédé de dépôt ou d'un procédé de mise en solution.

12. Diode organique électroluminescente selon la revendication 8, dans laquelle la diode organique électroluminescente est utilisée pour un dispositif choisi parmi un dispositif d'affichage plat, un dispositif d'affichage flexible, un dispositif d'éclairage plat monochrome ou à niveaux de gris, et un dispositif d'éclairage flexible monochrome ou à niveaux de gris.
